# FASCICULE DE BREVET EUROPEEN

(11) **EP 3 149 027 B1**
(45) Date de publication et mention de la délivrance du brevet: **24.12.2025**
(21) Numéro de dépôt: 15720445.4
(22) Date de dépôt: 27.03.2015
(51) Int. Cl.: C07K 14/415, C12N 15/82, C12N 9/90

(54) **AUGMENTATION DE LA RECOMBINAISON MÉIOTIQUE CHEZ LES PLANTES PAR INHIBITION D'UNE PROTÉINE RECQ4 OU TOP3A DU COMPLEXE RTR**
ERHÖHUNG IN MEIOTISCHER REKOMBINATION IN PFLANZEN DURCH HEMMUNG EINES RECQ4- ODER TOP3A-PROTEINS DES RTR-KOMPLEXES
INCREASE IN MEIOTIC RECOMBINATION IN PLANTS BY INHIBITING AN RECQ4 OR TOP3A PROTEIN OF THE RTR COMPLEX

(30) Priorité: 30.05.2014 FR 1454944
(43) Date de publication de la demande: 05.04.2017
(73) Titulaire: Institut national de recherche pour l'agriculture, l'alimentation et l'environnement, 75007 Paris (FR)
(72) Inventeur: MERCIER, Raphaël, 78340 Les Clayes sous Bois (FR); SEGUELA-ARNAUD, Mathilde, 78390 Bois d'Arcy (FR); CRISMANI, Wayne, West Des Moines, Iowa 50265 (US)
(74) Mandataire: Gevers & Orès
(86) Numéro de dépôt international: PCT/IB2015/052276
(87) Numéro de publication internationale: WO 2015/181647

(56) Documents cités:
- WO-A1-2013/038376
- HARTUNG FRANK ET AL: "Two closely related RecQ helicases have antagonistic roles in homologous recombination and DNA repair in Arabidopsis thaliana", PROCEEDINGS OF THE NATIONAL ACADEMY OF SCIENCES OF THE UNITED STATES OF AMERICA, vol. 104, no. 47, November 2007 (2007-11-01), pages 18836 - 18841, XP002734472, ISSN: 0027-8424
- BAGHERIEH-NAJJAR MOHAMMAD B ET AL: "Arabidopsis RecQl4A suppresses homologous recombination and modulates DNA damage responses", THE PLANT JOURNAL, BLACKWELL SCIENTIFIC PUBLICATIONS, OXFORD, GB, vol. 43, no. 6, 1 September 2005 (2005-09-01), pages 789 - 798, XP002568867, ISSN: 0960-7412, [retrieved on 20050816], DOI: 10.1111/J.1365-313X.2005.02501.X
- HARTUNG F ET AL: "The RecQ gene family in plants", JOURNAL OF PLANT PHYSIOLOGY, URBAN UND FISCHER VERLAG, DE, vol. 163, no. 3, 10 February 2006 (2006-02-10), pages 287 - 296, XP028020528, ISSN: 0176-1617, [retrieved on 20060210], DOI: 10.1016/J.JPLPH.2005.10.013
- WAYNE CRISMANI ET AL: "What limits meiotic crossovers?", CELL CYCLE, LANDES BIOSCIENCE, US, vol. 11, no. 19, 1 October 2012 (2012-10-01), pages 3527 - 3528, XP002721863, ISSN: 1538-4101, DOI: 10.4161/CC.21963

## Description

La présente invention est relative à une méthode pour augmenter la recombinaison méiotique chez les plantes.

La recombinaison méiotique est un échange d'ADN entre chromosomes homologues au cours de la méiose ; elle intervient pendant la prophase de la première division méiotique. Un des produits de la recombinaison est le crossing-over (ou crossover), qui entraine un échange réciproque de continuité entre deux chromatides homologues. Cette prophase (prophase I) comprend 5 stades successifs : leptotène, zygotène, pachytène, diplotène, et diacinèse. Au leptotène les chromosomes s'individualisent, chaque chromosome étant formé de deux chromatides sœurs issues de la duplication intervenue préalablement à la prophase. Durant le zygotène, les chromosomes homologues s'apparient, formant une structure appelée « bivalent » qui contient quatre chromatides, deux chromatides sœurs maternelles et deux chromatides sœurs paternelles, homologues des chromatides maternelles. Au pachytène les chromosomes sont totalement appariés, et des nodules de recombinaison se forment entre les chromatides homologues étroitement reliées entre elles par le complexe synaptonémal (SC) ; au diplotène, le SC se dissocie progressivement, et les chromosomes homologues commencent à se séparer, mais restent joints au niveau des chiasmas, qui correspondent aux sites de crossing-overs (COs). Les chromosomes se condensent au cours de la diacinèse ; les chiasmas subsistent jusqu'à la métaphase I pendant laquelle ils maintiennent l'appariement des bivalents de part et d'autre de la plaque équatoriale.

La recombinaison méiotique est déclenchée par la formation de cassures double-brin (CDB) dans l'une ou l'autre des chromatides homologues, et résulte de la réparation de ces cassures, utilisant comme matrice une chromatide du chromosome homologue.

La recombinaison méiotique a pour conséquence de provoquer un réassortiment des allèles d'origine paternelle et maternelle dans le génome, ce qui contribue à générer de la diversité génétique. Elle présente donc un intérêt particulier dans les programmes d'amélioration des plantes (Wijnker & de Jong, Trends in Plant Science, 2008, 13, 640-646 ; Crismani et al., Journal of Experimental Botany, 2013, 64, 55-65). Notamment, la possibilité d'augmenter le taux de recombinaison peut permettre d'obtenir de nouvelles combinaisons de caractéristiques ; elle peut permettre aussi de faciliter l'introgression de gènes d'intérêt d'une lignée à l'autre, ainsi que la cartographie génétique et le clonage positionnel de gènes d'intérêt.

Diverses méthodes pour contrôler la recombinaison méiotique ont été proposées, basées sur la sur-expression ou l'extinction de l'un ou l'autre des très nombreux gènes identifiés comme étant impliqués ou potentiellement impliqués dans ce mécanisme. Par exemple, la Demande PCT WO/0208432 propose la surexpression de la protéine RAD51, qui est impliquée dans la recombinaison homologue, pour stimuler la recombinaison méiotique ; la Demande US 2004/023388 propose de surexprimer un activateur de la recombinaison méiotique choisi parmi : SPO11, MRE11, RAD50, XRS2/NBS1, DMC1, RAD51, RPA, MSH4, MSHS, MLH1, RAD52, RAD54, TID1, RAD5S, RADS7, RADS9, une résolvase, une protéine liant l'ADN simple brin, une protéine impliquée dans le remodelage de la chromatine, ou une protéine du complexe synaptonémal, pour augmenter la fréquence de recombinaison entre chromatides homologues ; la Demande PCT WO 200/4016795 propose d'augmenter la recombinaison entre chromosomes homologues en exprimant une protéine SPO11 fusionnée à un domaine de liaison à l'ADN ; la Demande PCT WO 03/104451 propose d'augmenter le potentiel de recombinaison entre des chromosomes homologues par la surexpression d'une protéine (MutS) impliquée dans la réparation des mésappariements ; La Demande PCT WO 2010/071418 propose de réduire l'expression ou l'activité de la protéine RecQl5 (RECQ5).

Cependant, dans la plupart des cas, l'effet de ces gènes candidats sur la formation de COs méiotiques *in planta* n'a pas été confirmé, et il est donc nécessaire d'identifier d'autres gènes intervenant dans ce phénomène.

L'un des facteurs connus pour agir sur le taux de recombinaison méiotique est le phénomène d'interférence: la formation d'un CO à un endroit du chromosome inhibe la formation d'autres COs à proximité. Toutefois, il a été montré qu'il existait en fait 2 voies distinctes de formation des COs méiotiques (Hollingsworth & Brill, Genes Dev., 2004, 18,117-125; Berchowitz & Copenhaver, Current genomics, 2010, 11, 91-102). La première génère des COs interférents dénommés COs de type I (COI), et fait intervenir un ensemble de gènes collectivement désignés sous les appellations de gènes *ZMM* (*ZIP1, ZIP2*/*SHOC1, ZIP3, HEI10, ZIP4, MER3, MSH4, MSH5, PTD)* ainsi que les protéines *MLH1* et *MLH3 ;* la seconde voie génère des COs non-interférents, dénommés COs de type II (COII), et dépend du gène *MUS81.*

L'utilisation de l'une et/ou l'autre de ces deux voies est variable d'un organisme à l'autre. Chez les végétaux supérieurs, représentés par la plante modèle *Arabidopsis thaliana,* les 2 voies coexistent, celle des COs de type I étant prédominante ; il a été observé que l'inactivation des gènes *AtMSH4, AtMSH5, AtMER3, SHOC1, PTD, AtHEI10* ou *AtZIP4* induisait jusqu'à 85% de réduction de la fréquence des COs (Higgins et al., Genes Dev., 2004, 18, 2557-2570; Mercier et al., Curr. Biol., 2005, 15, 692-701; Chelysheva et al., PLoS Genet. 2007, 3, e83 ; Higgins et al., Plant J., 2008, 55, 28-39 ; Macaisne et al., Current Biology, 2008, 18, 1432-1437; Wijeratne et al., Molecular Biology of the Cell, 2006, 17, 1331-43; Chelysheva et al., PLoS Genetics, 2012, 8, e1002799). Certains chromosomes homologues ne sont de ce fait plus appariés sous forme de bivalents, mais apparaissent sous forme d'univalents. Cette diminution du nombre de bivalents s'accompagne d'une forte réduction de la fertilité.

Précédemment, les Inventeurs ont identifié un gène, dénommé *FANCM* (pour *« Fanconi Anemia Complementation Group M* ») dont l'inhibition compensait les effets de celle d'*AtMSH5,* de *SHOC1,* ou d*'AtZIP4* et permettait d'augmenter le nombre de COs méiotiques (Demande PCT WO 2013/038376 ; Crismani et al., Science 2012, 336, 6088, 1588-90).

En poursuivant leurs recherches, les Inventeurs ont maintenant identifié deux autres gènes dont l'inhibition produit des effets similaires à celle de *FANCM.*

Il s'agit des gènes *TOP3A* (pour ADN TOPOISOMERASE III alpha) et *RECQ4,* codant pour deux des protéines du complexe RTR.

Le complexe RTR est un complexe conservé, constitué d'une hélicase de la famille RecQ, d'une ADN Topoisomérase III (Topoisomérase de type I, sous-type IA) et d'une protéine structurale de la famille RMI1. Ce complexe, qui est impliqué dans la résolution des intermédiaires de recombinaison de l'ADN chez tous les eucaryotes, est essentiel pour le maintien de l'intégrité du génome(Mankouri et Hickson, Trends Biochem Sci., 2007, 32, 538-46).

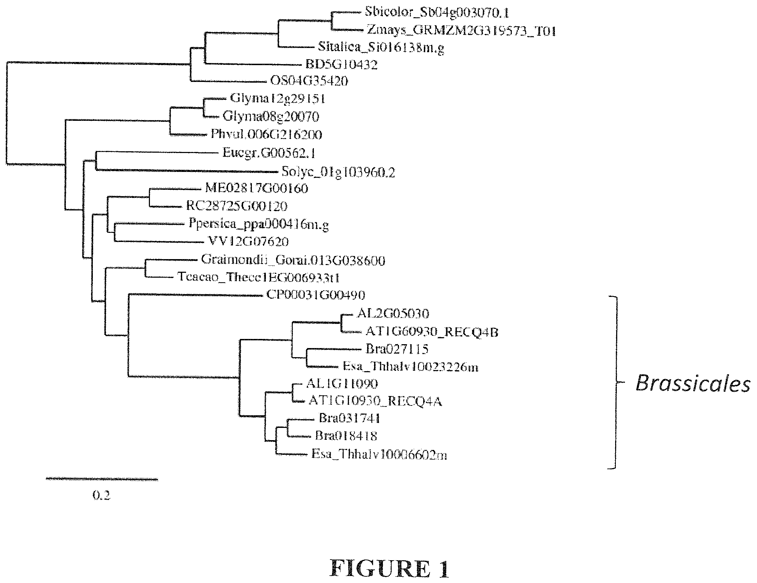

Les hélicases de la famille RecQ sont des protéines impliquées dans le maintien et la stabilité du génome chez tous les organismes tels que les bactéries, les levures, les animaux et les plantes. Chez les eucaryotes, le nombre de gènes RecQ (ou *RECQ*) et la structures des protéines RecQ (ou RECQ) varient énormément entre les organismes. Chez *Arabidopsis thaliana,* on dénombre 7 gènes *RECQ* dont *AtRECQ4A* qui code pour une protéine homologue à l'hélicase RecQ d'*Escherichia coli,* Sgs1 de levure et BLM humaine (Hartung F. et Puchta, H., J. Plant. Physiol., 2006, 163, 287-296 ; Hartung et al., Nucleic Acids Res., 2000, 28, 4275-4282 ; Bagherieh-Najjar et al., The Plant Journal, 2005, 43, 789-798; Crismani et al., Cell Cycle, 2012, 11, 3527-3528). Le gène *AtRECQ4B,* un paralogue d*'AtRECQ4A* issu probablement d'une duplication génique survenue uniquement chez les Brassicacae, code pour une protéine qui présente 70 % d'identité avec AtRECQ4A (Figure 1). Les protéines AtRECQ4A et AtRECQ4B comprennent 3 domaines conservés ; un domaine hélicase, qui est le plus important, et comprend huit motifs (0, I, Ia, II, III, IV, V et VI), d'une longueur totale d'environ 300 à 450 acides aminés, contenant les séquences nécessaires pour la liaison de l'ATP, l'hydrolyse et le déroulement de l'ADN (NCBI cd00079 et cd00046) ; un domaine RQC (smart00956), conservé chez presque toutes les protéines RECQ ; un domaine HRDC (pfam00570), conservé chez plus de 50 % des protéines RECQ et également en C-terminal de la RNase D.

Alors que chez la levure *S. cerevisiae,* la recombinaison méiotique est augmentée en l'absence de l'hélicase Sgs1 d'un facteur 1.17-1.6 (Jessop et al., PLoS Genetics, 2006, 2, e155 ; Oh et al., Cell, 2007, 130(2), 259-272), la mutation d*'AtRECQ4A* (*recq4a-5*/*SALK_069672*) ou *AtRECQ4B* est décrite comme ne produisant aucun effet significatif sur la formation de COs méiotiques (Higgins et al., The Plant Journal, 2011, 65, 492-502). Il a notamment été observé que la mutation de d*'AtRECQ4A* (*recq4a-*5/SALK_069672) ou d*'AtRECQ4B* était incapable de restaurer la fertilité et la formation de bivalents chez un mutant *msh4* de la souche Columbia. En revanche, il a été suggéré que RECQ4A jouait un rôle dans le maintien des télomères, un rôle dans l'intégrité du génome, auparavant inconnu pour une protéine de la famille RecQ.

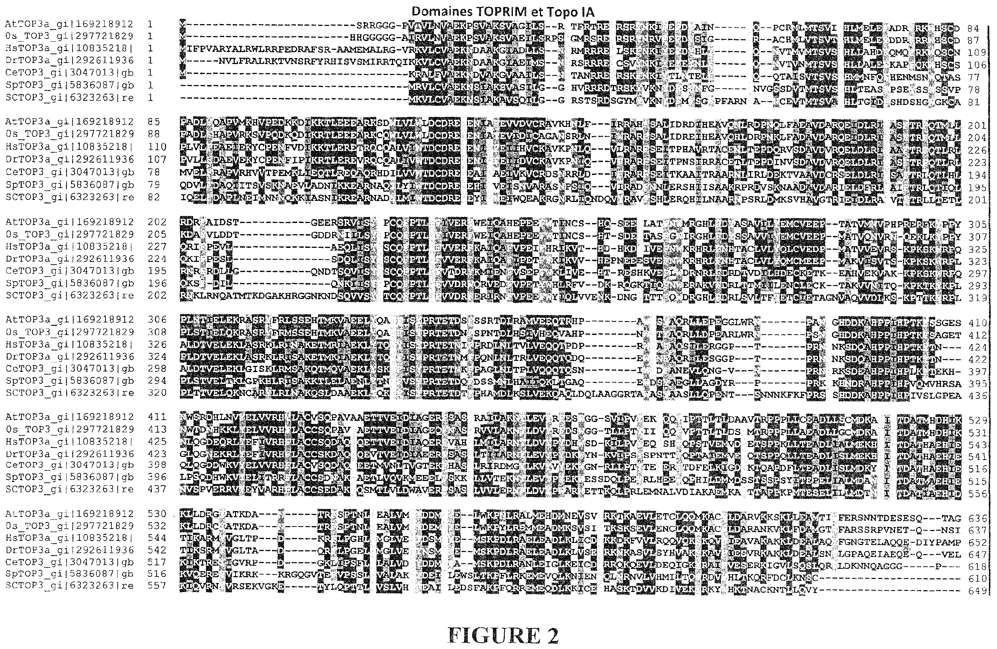

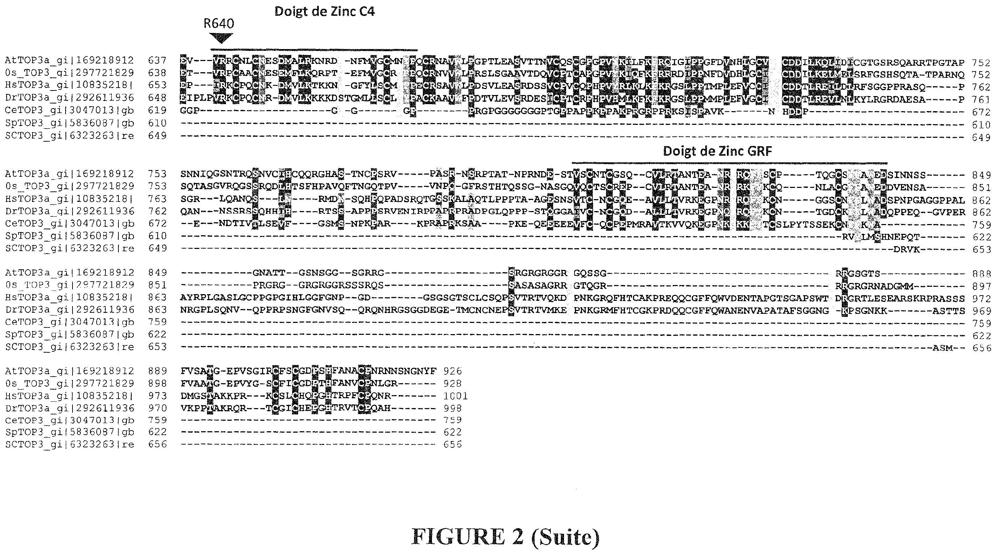

La protéine AtTOP3A (TOP3a, TOP3, Top3a, Top3 alpha, TOP3 alpha ou TOP3α, pour ADN TOPOISOMERASE III alpha ou ADN TOPOISOMERASE 3-alpha) contient 4 domaines conservés: dans sa région N-terminale, un domaine TOPRIM (NCBI cd03362) et un domaine ADN Topoisomerase de sous-type IA (NCBI cd00186), tous deux conservés chez tous les homologues de TOP3 ; dans sa région C-terminale, deux domaines en doigt de zinc (pfam01396 et pfam06839) , qui sont conservés chez les plantes et les animaux mais pas chez les levures (Figure 2).

Deux allèles mutants de *top3a* (*top3a-1* and *top3a-2*) ont précédemment été décrits chez *Arabidopsis* (Hartung et al., PLoS Genet., 2008, 4, e1000285, Hartung et al., PNAS 2007, 104, 47, 18836-41 ). La mutation *top3a-1* est vraisemblablement nulle étant donné qu'elle conduit à une létalité précoce au cours du développement. Le second allèle, *top3a-2,* est un mutant hypo morphe qui est viable mais montre des défauts de croissance somatique, et est complètement stérile (Hartung et al., PLoS Genet., 2008, 4, e1000285). Les phénotypes sévères associés à l'inactivation de *TOP3A* chez *Arabidopsis* et des espèces variées (Goodwin et al., Nucleic Acids Res., 1999, 27, 4050-8; Kim et al., Nucleic Acids Res., 2000, 28, 2012-7; Li et Wang, , Proc. Natl. Acad. Sci. U. S. A., 1998, 95, 1010-3 Plank et al., J. Biol. Chem., 2005, 280, 3564-73) soulignent un rôle essentiel de TOP3A dans la résolution des intermédiaires mitotiques et méiotiques de réparation de l'ADN mais ne suggèrent pas une activité anti-CO méiotique de cette protéine.

Au contraire, comme démontré dans la présente invention, l'inhibition des gènes *AtRECQ4A et AtRECQ4B* ou *TOP3A* augmente le nombre de COs méiotiques, et ce non seulement chez les mutants *zmm,* mais également chez les plantes de type sauvage pour les gènes ZMM.

En effet, les inventeurs ont montré que l'inhibition d*'AtRECQ4A* et d*'AtRECQ4B* compense les effets de celle d*'AtMSH4* ou d*'AtZIP2*/*SHOC1* et permet d'augmenter le nombre de COs méiotiques et de restaurer la fertilité chez les mutants *zmm Atmsh4-*/*-* et *shoc1-*/*-.*

L'inhibition de *TOP3A* compense les effets de celle d*'AtHEI10* ou d'*AtMSH5* et permet d'augmenter le nombre de COs méiotiques et de restaurer la fertilité chez les mutants *zmm Athei10-*/*-et Atmsh5-*/*- .*

Les Inventeurs ont également découvert que les effets de l'inhibition du gène *TOP3A* ou *RECQ4* sur l'augmentation du nombre de COs méiotiques se produisaient non seulement chez les mutants *zmm,* mais également chez les plantes possédant des gènes *ZMM* fonctionnels.

Les protéines FIDG appartiennent à la famille des AAA-ATPases (ATPases Associated with various Activities), et plus particulièrement au sous-groupe 7 (Beyer, Protein Sci., 1997, 6, 2043-58 ; Frickey & Lupas, J. Struct. Biol., 2004,146, 2-10,). Un seul représentant de cette famille a été identifié dans le génome de la plupart des plantes. Les protéines FIDG présentent deux domaines protéiques conservés : un domaine AAA-ATPase, avec un rôle putatif d'hydrolyse de l'ATP ; ainsi qu'un domaine VSP4, connu pour jouer un rôle important dans la liaison aux microtubules. Ces deux domaines sont respectivement répertoriés sous les références PF00004 et PF09336 dans la base de données PFAM (Punta et al., Nucleic Acids Res., 2012 , Database Issue 40:D290-D301).

Les protéines FIDG sont décrites comme des enzymes de sectionnement des microtubules, impliquées dans la régulation du nombre et de la taille de microtubules chez des nombreuses espèces animales et végétales (*C. elegans* (Yakushiji et al., FEBS Lett., 2004, 578, 191-7) ; *D. melanogaster* (Zhang et al., J. Cell. Biol., 2007, 177, 231-42); *H. sapiens* (Mukherjee et al., Cell Cycle, 2012, 11, 2359-66); A. *thaliana* (Stoppin-Mellet et al., Biochem J., 2002, 365, 337-42)).

Les Inventeurs ont en outre découvert que lorsque l'inhibition du gène *TOP3A* ou *RECQ4* était combinée à celle du gène *FANCM ou FIDG,* le nombre de COs méiotiques était encore augmenté par rapport à ceux observés lorsque ces gènes étaient inactivés séparément.

L'invention est telle que définie dans les revendications 1 à 13.

L'invention concerne un procédé pour augmenter la fréquence des crossovers méiotiques chez une plante, caractérisé en ce qu'il comprend l'inhibition dans ladite plante de l'expression ou de la fonction, d'au moins une protéine du complexe RTR dénommée RECQ4, par mutagénèse du gène codant ladite protéine ou de son promoteur, par extinction du gène codant ladite protéine ou par un inhibiteur se liant spécifiquement à un domaine fonctionnel de ladite protéine, ladite protéine RECQ4 ayant au moins 40 % d'identité de séquence avec la protéine RECQ4 de séquence SEQ ID NO: 1 et comprenant en outre une région ayant au moins 60% d'identité de séquence avec la région qui s'étend des positions 407 à 959 de la séquence SEQ ID NO : 1.

Dans un mode de réalisation, l'invention concerne un procédé tel que décrit précédemment, caractérisé en ce qu'il comprend en outre l'inhibition dans ladite plante de l'expression ou de la fonction d'une protéine du complexe RTR dénommée TOP3A, par mutagénèse du gène codant ladite protéine ou de son promoteur, par extinction du gène codant ladite protéine ou par un inhibiteur se liant spécifiquement à un domaine fonctionnel de ladite protéine, ladite protéine TOP3A ayant au moins 50 % d'identité de séquence avec la protéine TOP3A de séquence SEQ ID NO: 2.

Dans un mode de réalisation, l'invention concerne un procédé tel que décrit précédemment, caractérisé en ce qu'il comprend en outre l'inhibition dans ladite plante de l'expression ou de la fonction , d'au moins une protéine choisie parmi :
- une protéine ci-après dénommée FIDG, ladite protéine ayant au moins 45% d'identité de séquence, ou au moins 60% de similarité de séquence avec la protéine AtFIDG de séquence SEQ ID NO: 46 et contenant un domaine AAA-ATPase et un domaine VSP4, et
- une protéine ci-après dénommée FANCM, ladite protéine ayant au moins 30% d'identité de séquence, ou au moins 45% de similarité de séquence avec la protéine AtFANCM de séquence SEQ ID NO: 45, et contenant un domaine hélicase DEXDc et un domaine hélicase HELICc,
par mutagénèse du gène codant ladite protéine ou de son promoteur, par extinction du gène codant ladite protéine ou par un inhibiteur se liant spécifiquement à un domaine fonctionnel de ladite protéine.

La protéine RECQ4 peut en outre comprendre une région ayant au moins 60%, et par ordre de préférence croissante au moins 65, 70, 75, 80, 85, 90, 95 ou 98% d'identité de séquence avec la région qui s'étend des positions 407 à 959 de la séquence SEQ ID NO: 1.

Alternativement, la protéine RECQ4 peut être définie comme une protéine comprenant une région ayant au moins 60%, et par ordre de préférence croissante au moins 65, 70, 75, 80, 85, 90, 95 ou 98% d'identité de séquence avec la région qui s'étend des positions 407 à 959 de la séquence SEQ ID NO: 1.

Les identités de séquences des protéines RECQ4 et TOP3 sont calculées sur la totalité de la longueur de la plus longue des deux protéines que l'on compare après alignement à l'aide de T-Coffee (v6.85) avec les paramètres par défaut (http://toolkit.tuebingen.mpg.de/t_coffee). Le pourcentage d'identité des protéines RECQ4 et TOP3 est obtenu à partir de cet alignement en utilisant Bioedit 7.2.5 (Hall, T.A. 1999. BioEdit: a user-friendly biological sequence alignment editor and analysis program for Windows 95/98/NT. Nucl. Acids. Symp. Ser. 41:95-98). Le pourcentage d'identité de la région de la protéine RECQ4 est calculé sur la séquence des positions 407-959 de la séquence SEQ ID NO : 1 après alignement de la protéine RECQ4 avec la séquence SEQ ID NO : 1.

Les séquences des gènes et protéines RECQ4 et TOP3A sont disponibles sur les bases de données publiques, telles que de façon non-limitative la base de données PLAZA (http://bioinformatics.psb.ugent.be/plaza/) et phytozome http://www.phytozome.net/ (phytosome v9.1).

Au sens de la présente invention, l'expression protéine RECQ4 correspond aux protéines RECQ4A et RECQ4B chez les plantes de la famille des Brassicacae (*Brassica sp.*) qui possèdent deux gènes RECQ4 (*RECQ4A* et *RECQ4B*), et à la protéine RECQ4 chez les plantes autres que les Brassicacae, qui ne possèdent qu'un seul gène *RECQ4.* Toutefois, chez certaines lignées de Brassicacae dans lesquelles l'une des protéines RECQ4 n'est pas fonctionnelle, seule la protéine RECQ4 qui est fonctionnelle est inhibée.

La séquence SEQ ID NO : 1 représente la séquence de la protéine RECQ4A *d'Arabidopsis thaliana* (AT1G10930 dans la base de données PLAZA). Les séquences d'orthologues de RECQA et de paralogues RECQB chez différentes espèces de plantes à fleurs sont indiquées dans le Tableau ci-après :

**Tableau I : Séquences de RECQ4**

| **Plante** | **Numéro d'accès*** | **SEQ ID NO :** |
|---|---|---|
| *Arabidopsis thaliana* | AT1G10930 | **1** |
| *Arabidopsis lyrata* | AL1G11090 | **3** |
| *Arabidopsis lyrata* | AL2G05030 | **4** |
| *Arabidopsis thaliana* | AT1G60930** | **5** |
| *Brachypodium distachyon* | BD5G10432 | **6** |
| *Brassica rapa* | Bra031741 | **7** |
| *Brassica rapa* | Bra027115 | **8** |
| *Brassica rapa* | Bra018418 | **9** |
| *Carica papaya* | CP00031G00490 | **10** |
| *Thellungiella halophila* | Thhalv10006602m | **11** |
| *Thellungiella halophila* | Thhalv10023226m | **12** |
| *Eucalyptus grandis* | Eucgr.G00562.1 | **13** |
| *Glycine max* | Glyma12g29151 | **14** |
| *Glycine max* | Glyma08g20070 | **15** |
| *Gossypium raimondii* | Gorai.013G038600 | **16** |
| *Manihot esculenta* | ME02817G00160 | **17** |
| *Oryza sativa* | OS04G35420 | **18** |
| *Phaseolus vulgaris* | Phvul.006G216200 | **19** |
| *Prunus persica* | ppa000416m.g | **20** |
| *Ricinus communis* | RC28725G00120 | **21** |
| *Sorghum bicolor* | Sb04g003070.1 | **22** |
| *Setaria italica* | Si016138m.g | **23** |
| *Solanum lycopersicum* | 01g103960.2 | **24** |
| *Theobroma cacao* | Thecc1EG006933t1 | **25** |
| *Vitis vinifera* | VV12G07620 | **26** |

| | | |
|---|---|---|
| *dans la base de données PLAZA ou phytozome **RECQ4B | | |

Conformément à l'invention, ladite protéine RECQ4 comprend 3 domaines conservés : un domaine hélicase, qui est le plus important, et comprend huit motifs (0, I, Ia, II, III, IV, V et VI), d'une longueur totale d'environ 300 à 450 acides aminés, contenant les séquences nécessaires pour la liaison de l'ATP, l'hydrolyse et le déroulement de l'ADN (NCBI cd00079 et cd00046) ; un domaine RQC (smart00956) ; un domaine HRDC (pfam00570).

La séquence SEQ ID NO : 2 représente la séquence de la protéine TOP3A d'*Arabidopsis thaliana* (AT5G63920 dans la base de données PLAZA). Les séquences d'orthologues chez différentes espèces de plantes à fleurs sont indiquées dans le Tableau ci-après :

**Tableau II : Séquences de TOP3A**

| **Plante** | **Numéro d'accès*** | **SEQ ID NO :** |
|---|---|---|
| *Arabidopsis thaliana* | AT5G63920 | **2** |
| *Arabidopsis lyrata* | AL8G30570 | **27** |
| *Brachypodium distachyon* | BD1G74120 | **28** |
| *Fragaria vesca* | FV0G21450 | **29** |
| *Oryza sativa* | OS03G06900 | **30** |
| *Populus trichocarpa* | PT05G06620 | **31** |
| *Ricinus communis* | RC30149G00070 | **32** |
| *Sorghum bicolor* | SB01G046210 | **33** |
| *Zea mays* | ZM01G04670 | **34** |
| *Thellungiella halophila* | Thhalv10003618m.g | **35** |
| *Capsella rubella* | Carubv10025845m | **36** |
| *Brassica rapa* | Bra031944 | **37** |
| *Gossypium raimondii* | Gorai.007G066700. | **38** |
| *Theobroma cacao* | Thecc1EG001553t1 | **39** |
| *Eucalyptus grandis* | Eucgr.H05141.1 | **40** |
| *Glycine max* | Glyma06g16175.1 | **41** |
| *Mimulus guttatus* | mgv1a000984m.g | **42** |
| *Aquilegia caerula* | Aquca_069_00036.1 | **43** |
| *Setaria italica* | Si034218m.g | **44** |

| | | |
|---|---|---|
| *dans la base de données PLAZA ou phytozome | | |

Conformément à l'invention, la protéine TOP3A comprend 4 domaines conservés: dans sa région N-terminale, un domaine TOPRIM (NCBI cd03362) et un domaine ADN Topoisomerase de sous-type IA (NCBI cd00186) ; dans sa région C-terminale, deux domaines en doigt de zinc (pfam01396 et pfam06839 ; Figure 2). Le premier domaine en doigt de zinc est situé dans la région de ladite protéine TOP3A qui s'étend des positions 639 à 677 de la séquence SEQ ID NO : 2. Le deuxième domaine en doigt de zinc est situé dans la région de ladite protéine TOP3A qui s'étend des positions 804 à 844 de la séquence SEQ ID NO : 2.

L'invention englobe l'inhibition simultanée des protéines RECQ4 et/ou TOP3A.

L'inhibition de la protéine RECQ4 et/ou TOP3A est obtenue par l'abolition, le blocage ou l'inhibition de l'expression du gène *RECQ4* ou *TOP3A* ou bien de la fonction de ladite protéine RECQ4 ou TOP3A. En outre, l'inhibition de la protéine TOP3A dans la plante est effectuée de façon à ce que la plante exprime nécessairement, à partir du gène *TOP3A* muté ou d'un transgène, une protéine TOP3A mutée comprenant une mutation dans sa région C-terminale qui inhibe au moins l'un des deux domaines en doigt de zinc alors que sa région N-terminale comprenant les domaines TOPRIM et ADN topoisomerase IA est intacte.

L'inhibition peut notamment être obtenue par mutagenèse du gène *RECQ4* ou *TOP3A.* Par exemple, une mutation dans la séquence codante peut induire, selon la nature de la mutation, l'expression d'une protéine inactive, ou d'une protéine à activité réduite; une mutation au niveau d'un site d'épissage peut également altérer ou abolir la fonction de la protéine ; une mutation dans la séquence promotrice peut induire l'absence d'expression de ladite protéine, ou la diminution de son expression.

La mutagenèse peut être effectuée par exemple par la délétion de tout ou partie de la séquence codante ou du promoteur de *RECQ4* ou *TOP3A,* ou par l'insertion d'une séquence exogène, par exemple un transposon ou un ADN-T, au sein de ladite séquence codante ou dudit promoteur. Elle peut également être effectuée en induisant des mutations ponctuelles, par exemple par mutagénèse EMS, par radiations, ou par mutagénèse dirigée, par exemple à l'aide de nucléases TALENs (Transcription Activator-Like Effector Nucleases), des systèmes CRISPR-CAS ou de nucléases à doigt de zinc (CHRISTIAN et al., Genetics, 186, 757-61, 2010; CURTIN et al., Plant Gen., 5, 42-50, 2012).

Les allèles mutés peuvent être détectés par exemple par PCR, en utilisant des amorces spécifiques du gène *RECQ4 ou TOP3A.*

Différentes méthodes de mutagénèse et de criblage à haut débit sont décrites dans l'art antérieur. A titre d'exemples, on peut citer des méthodes de type « TILLING » (Targeting Induced Local Lesions In Genome), décrites par McCallum et al., Plant Physiol., 2000, 123, 439-442). L'absence de fonctionnalité de RECQ4 ou TOP3A chez les mutants peut être vérifiée sur la base des caractéristiques phénotypiques de leur descendance ; les plantes homozygotes pour une mutation inactivant le gène *RECQ4* ou *TOP3A* ont un taux de COs méiotiques supérieur à celui des plantes sauvages (ne portant pas la mutation dans le gène *RECQ4* ou *TOP3A*) dont elles sont issues. Généralement ce taux de COs méiotiques est supérieur d'au moins 50%, de préférence au moins 2 fois supérieur à celui des plantes sauvages dont elles sont issues.

Selon un mode de mise en œuvre avantageux de ladite méthode, elle comprend l'introduction, dans un allèle du gène *TOP3A,* d'une mutation qui résulte dans l'inhibition d'au moins un domaine en doigt de zinc de la protéine TOP3A, ledit domaine étant situé dans la région de ladite protéine qui s'étend des positions 639 à 677 ou 804 à 844 de la séquence SEQ ID NO : 2. La mutation préserve les domaines TOPRIM et ADN topoisomerase IA mais inhibe, et de préférence inactive, au moins l'un des deux domaines en doigt de zinc. Il s'agit notamment d'une insertion ou d'une délétion dans la région C-terminale de TOP3A comprenant lesdits domaines. De préférence, ladite mutation est une délétion de la séquence C-terminale de ladite protéine TOP3A, à partir d'un des résidus de ladite protéine situé des positions 610 à 803 de la séquence SEQ ID NO : 2, de manière préférée à partir du résidu de ladite protéine situé en position 640 de la séquence SEQ ID NO : 2 (production d'une protéine TOP3A tronquée en C-terminal).

Alternativement, l'inhibition de la protéine RECQ4 ou TOP3A est obtenue par extinction (silencing) du gène *RECQ4* ou *TOP3A.* Différentes techniques d'extinction de gènes dans les plantes sont connues en elles-mêmes (pour revue voir par exemple : Watson & Grierson, Transgenic Plants : Fundamentals and Applications (Hiatt, A, ed) New York : Marcel Dekker, 255-281, 1992 ; Chicas & Macino, EMBO reports, 2001, 21, 992-996 ; Puchta, H et Fauser, F, Int. J. Dev. Biol., 2013, 57, 629-37 ; Ali et al., GM crops, 2010, 1, 207-213). On peut citer l'inhibition antisens ou co-suppression, décrites par exemple dans les Brevets US°5190065 et US°5283323. Il est également possible d'utiliser des ribozymes ciblant l'ARNm de la protéine RECQ4 ou TOP3A.

De préférence, l'extinction du gène *RECQ4* ou *TOP3A* est induite par interférence ARN ciblant ledit gène.

Dans un mode de réalisation, l'invention concerne un procédé tel que décrit précédemment, caractérisé en ce que l'inhibition de la protéine RECQ4 et/ou TOP3A et le cas échéant de la protéine FIDG et/ou FANCM, est obtenue par extinction du gène codant pour ladite protéine en exprimant dans ladite plante un ARN interférent ciblant ledit gène.

Un ARN interférent (ARNi) est un petit ARN qui peut éteindre un gène cible de manière séquence spécifique. Les ARN interférents comprennent en particulier les « petits ARN interférents » (siRNA) et les micro-ARN (miRNA).

Initialement, les constructions d'ADN pour exprimer des ARN interférents dans les plantes contenaient un fragment de 100 pb ou plus (généralement de 100 à 800 pb) de l'ADNc du gène ciblé, sous contrôle transcriptionnel d'un promoteur approprié. Des constructions largement utilisées sont celles qui peuvent produire un transcrit d'ARN en épingle à cheveux (hpRNA). Dans ces constructions, le fragment du gène cible est inversement répété, avec généralement une région d'espacement entre les répétitions (pour revue, cf. Watson et al., FEBS Letters, 2005, 579, 5982-5987). On peut également utiliser des micro-ARN artificiels (amiRNAs) dirigés contre le gène *RECQ4* ou *TOP3A* (Ossowski et al., The Plant Journal, 2008, 53, 674-690, ; Schwab et al., Methods Mol Biol., 2010, 592, 71-88; Wei et al., Funct Integr Genomics., 2009, 9, 499-511).

Pour inhiber la protéine TOP3A, la méthode de l'invention est avantageusement mise en œuvre avec l'une des plantes génétiquement modifiées suivantes :
(i) une plante mutante homozygote pour la mutation de *TOP3A,* telle que définie ci-dessus, qui résulte dans l'expression d'une protéine TOP3A mutée dans le domaine C-terminal, par exemple une protéine TOP3A tronquée, dont les domaines TOPRIM et Topoisomérase IA sont intactes mais l'un au moins ou les deux domaines en doigt de zinc sont inhibés, et de préférence inactivés,
(ii) une plante mutante et transgénique, homozygote pour une mutation qui inhibe le gène TOP3A et comprenant un transgène *TOP3A* codant pour une protéine TOP3A mutée en C-terminal, par exemple une protéine TOP3A tronquée, telle que définie ci-dessus, et
(iii) une plante double transgénique, comprenant un premier transgène codant pour un ARNi qui cible le gène *TOP3A* et un deuxième transgène comprenant un gène *TOP3A* recombinant codant pour une protéine TOP3A mutée, par exemple une protéine TOP3A tronquée en C-terminal, telle que définie ci-dessus ; le deuxième transgène n'étant pas sensible au RNAi produit par le premier transgène. Pour ce faire, on peut, utiliser, de façon non-limitative, un RNAi ciblant la région C-terminale de *TOP3A* L'inhibition de la protéine RECQ4 ou TOP3A peut également être obtenue par l'abolition, le blocage ou la diminution de la fonction de ladite protéine. Pour inhiber l'activité de ladite protéine ont peut utiliser des inhibiteurs qui se lient spécifiquement à un domaine fonctionnel de ladite protéine et bloquent son activité. Il s'agit notamment d'inhibiteurs protéiques, tels que des peptides ou des anticorps ou fragments fonctionnels d'anticorps, ou bien des petites molécules. A titre d'exemple non-limitatif on peut citer les petites molécules inhibitrices de RecQ telles que décrites dans Nguyen et al., Chemistry & Biology, 2013, 20, 1, 24, 55-62. De manière avantageuse, ces inhibiteurs sont appliqués sur les plantes.

La présente invention concerne également une cassette d'expression, caractérisée en ce qu'elle comprend une séquence d'ADN recombinant dont le transcrit est un ARN interférent ciblant le gène *RECQ4,* placée sous contrôle transcriptionnel d'un promoteur fonctionnel dans une cellule végétale, ledit gène *RECQ4* codant une protéine RECQ4 ayant au moins 40 % d'identité de séquence avec la protéine RECQ4 de séquence SEQ ID NO: 1 et comprenant en outre une région ayant au moins 60% d'identité de séquence avec la région qui s'étend des positions 407 à 959 de la séquence SEQ ID NO : 1.

Dans un mode de réalisation, l'invention concerne une cassette d'expression telle que décrite précédemment, caractérisée en ce qu'elle comprend en outre une séquence d'ADN recombinant codant pour une protéine TOP3A comprenant un domaine TOPRIM et un domaine Topoisomérase IA non-altérés et au moins un domaine en doigt de zinc qui est inactivé, placée sous contrôle transcriptionnel d'un promoteur fonctionnel dans une cellule végétale, ladite protéine TOP3A ayant au moins 50 % d'identité de séquence avec la protéine TOP3A de séquence SEQ ID NO: 2.

Dans un mode de réalisation, l'invention concerne une cassette d'expression telle que décrite précédemment, caractérisée en ce qu'elle comprend en outre une séquence d'ADN recombinant dont le transcrit est un ARN interférent ciblant le gène *FANCM* ou *FIDG,* placée sous contrôle transcriptionnel d'un promoteur fonctionnel dans une cellule végétale, ledit gène *FANCM* codant une protéine FANCM ayant au moins 30% d'identité de séquence, ou au moins 45% de similarité de séquence avec la protéine AtFANCM de séquence SEQ ID NO: 45, et contenant un domaine hélicase DEXDc et un domaine hélicase HELICc et ledit gène *FIDG* codant une protéine FIDG ayant au moins 45% d'identité de séquence, ou au moins 60% de similarité de séquence avec la protéine AtFIDG de séquence SEQ ID NO: 46 et contenant un domaine AAA-ATPase et un domaine VSP4.

La protéine TOP3A mutée peut notamment être exprimée à partir d'un promoteur de gène *TOP3A,* notamment de plante, tel que le promoteur du gène *TOP3A* de la plante transgénique dans laquelle ladite protéine TOP3A mutée en C-terminal est exprimée.

Un large choix de promoteurs appropriés pour l'expression de gènes hétérologues dans des cellules végétales ou des plantes est disponible dans l'art.

Ces promoteurs peuvent être obtenus par exemple à partir de plantes, de virus de plantes, ou de bactéries comme *Agrobacterium.* Ils incluent des promoteurs constitutifs, à savoir des promoteurs qui sont actifs dans la plupart des tissus et cellules et dans la plupart des conditions environnementales, ainsi que des promoteurs tissu-spécifiques ou cellule spécifiques, qui sont actifs uniquement ou principalement dans certains tissus ou certains types de cellules, et des promoteurs inductibles qui sont activés par des stimuli physiques ou chimiques.

Des exemples de promoteurs constitutifs qui sont couramment utilisés dans les cellules végétales sont le promoteur 35S du virus de la mosaïque du chou-fleur (CaMV) décrit par Kay et al. (Science, 1987, 236, 4805) ou ses dérivés, le promoteur du virus de la mosaïque des nervures de manioc (CsVMV) décrit dans la Demande Internationale WO 97/48819, le promoteur de l'ubiquitine du maïs ou le promoteur « Actine-Intron-actine » du riz (McElroy et al., Mol. Gen. Genet., 1991, 231, 150-160; GenBank numéro d'accès S 44221). Pour la mise en œuvre de la présente invention, on choisira un promoteur fonctionnel dans les méiocytes.

Dans le cadre de la présente invention on pourra aussi utiliser un promoteur spécifique de la méiose (c'est-à-dire actif exclusivement ou préférentiellement dans les cellules en méiose). A titre d'exemple non limitatif on peut citer le promoteur DMC1 (Klimyuk & Jones, Plant J., 1997, 11, 1-1.).

Les cassettes d'expression de l'invention comprennent généralement un terminateur transcriptionnel, par exemple le terminateur 3'NOS de la nopaline synthase (Depicker et al., J. Mol. Appl. Genet., 1982, 1, 561-573,), le terminateur 3'CaMV (Franck et al., Cell, 1980, 21, 285-294) ou le terminateur d'un gène *TOP3A,* notamment de plante tel que le terminateur du gène *TOP3A* de la plante transgénique dans laquelle ladite protéine TOP3A mutée est exprimée. Elles peuvent également comprendre d'autres éléments de régulation de la transcription tels que des activateurs.

Les constructions d'ADN recombinant conformes à l'invention peuvent comprendre plusieurs cassettes d'expressions codant respectivement pour l'un des ARNi ciblant le gène *RECQ4* ou *TOP3A* et une protéine TOP3A mutée en C-terminal, notamment deux cassettes d'expressions, l'une codant pour un ARNi ciblant le gène *TOP3A* et l'autre pour une protéine TOP3A mutée en C-terminal.

La présente invention concerne également un vecteur recombinant contenant une cassette d'expression telle que décrite précédemment.

Les constructions d'ADN recombinant conformes à l'invention englobent également des vecteurs recombinants contenant une ou plusieurs cassettes d'expression conformes à l'invention telles que définies ci-dessus. Ces vecteurs recombinants peuvent également inclure un ou plusieurs gènes marqueurs, qui permettent la sélection des cellules ou plantes transformées.

Le choix du vecteur le plus approprié dépend notamment de l'hôte prévu et de la méthode envisagée pour la transformation de l'hôte en question. De nombreuses méthodes pour la transformation génétique de cellules végétales ou de plantes sont disponibles dans l'art, pour de nombreuses espèces végétales, dicotylédones ou monocotylédones. A titre d'exemples non-limitatifs, on peut citer la transformation médiée par virus, la transformation par microinjection, par électroporation, la transformation par microprojectiles, la transformation par *Agrobacterium,* etc.

La présente invention concerne également une cellule-hôte transformée par un vecteur recombinant tel que décrit précédemment.

L'invention a également pour objet une cellule-hôte comprenant au moins une construction d'ADN recombinant conforme à l'invention. Ladite cellule peut comprendre deux constructions d'ADN recombinant, l'une codant pour un ARNi ciblant le gène *TOP3A* et l'autre pour une protéine TOP3A mutée en C-terminal. Ladite cellule-hôte peut être une cellule procaryote, par exemple une cellule d'*Agrobacterium,* ou une cellule eucaryote, par exemple une cellule végétale génétiquement transformés par au moins une construction d'ADN de l'invention. La construction peut être exprimée transitoirement, elle peut aussi être incorporée dans un réplicon extrachromosomique stable, ou intégrée dans le chromosome.

La présente invention concerne également une plante mutante caractérisée en ce qu'elle contient une mutation dans le gène *RECQ4* codant une protéine RECQ4 ayant au moins 40 % d'identité de séquence avec la protéine RECQ4 de séquence SEQ ID NO: 1 et comprenant en outre une région ayant au moins 60% d'identité de séquence avec la région qui s'étend des positions 407 à 959 de la séquence SEQ ID NO : 1, ladite mutation induisant l'inhibition de l'expression ou de la fonction de la protéine RECQ4 dans ladite plante et étant choisie parmi les mutations ponctuelles ou la délétion de tout ou partie de la séquence codante ou du promoteur dudit gène.

Dans un mode de réalisation, l'invention concerne une plante mutante telle que décrite précédemment, caractérisée en ce qu'elle contient en outre :
- une mutation dans le gène *TOP3A* codant une protéine TOP3A ayant au moins 50 % d'identité de séquence avec la protéine TOP3A de séquence SEQ ID NO: 2, le gène *FIDG* codant une protéine FIDG ayant au moins 45% d'identité de séquence, ou au moins 60% de similarité de séquence avec la protéine AtFIDG de séquence SEQ ID NO: 46 et contenant un domaine AAA-ATPase et un domaine VSP4, et/ou le gène *FANCM* codant une protéine FANCM ayant au moins 30% d'identité de séquence, ou au moins 45% de similarité de séquence avec la protéine AtFANCM de séquence SEQ ID NO: 45, et contenant un domaine hélicase DEXDc et un domaine hélicase HELICc, ladite mutation induisant l'inhibition de l'expression ou de la fonction de la protéine TOP3A, FIDG et/ou FANCM dans ladite plante ou au moins une séquence d'ADN recombinant dont le transcrit est un ARN interférent ciblant le gène *TOP3A, FIDG* ou *FANCM,* placée sous contrôle transcriptionnel d'un promoteur fonctionnel dans ladite plante ; et/ou
- une séquence d'ADN recombinant codant pour une protéine TOP3A ayant au moins 50 % d'identité de séquence avec la protéine TOP3A de séquence SEQ ID NO: 2, comprenant un domaine TOPRIM et un domaine Topoisomérase IA non-altérés et au moins un domaine en doigt de zinc qui est inactivé, placée sous contrôle transcriptionnel d'un promoteur fonctionnel dans ladite plante.

La présente invention concerne également une plante transgénique contenant un transgène comprenant une séquence d'ADN recombinant dont le transcrit est un ARN interférent ciblant le gène RECQ4, placée sous contrôle transcriptionnel d'un promoteur fonctionnel dans ladite plante, ledit gène *RECQ4* codant une protéine RECQ4 ayant au moins 40 % d'identité de séquence avec la protéine RECQ4 de séquence SEQ ID NO: 1 et comprenant en outre une région ayant au moins 60% d'identité de séquence avec la région qui s'étend des positions 407 à 959 de la séquence SEQ ID NO : 1.

Dans un mode de réalisation, l'invention concerne une plante transgénique telle que définie précédemment, caractérisée en ce qu'elle contient en outre :
- une séquence d'ADN recombinant dont le transcrit est un ARN interférent ciblant le gène *TOP3A* codant la protéine TOP3A ayant au moins 50 % d'identité de séquence avec la protéine TOP3A de séquence SEQ ID NO: 2, le gène *FIDG* codant une protéine FIDG ayant au moins 45% d'identité de séquence, ou au moins 60% de similarité de séquence avec la protéine AtFIDG de séquence SEQ ID NO: 46 et contenant un domaine AAA-ATPase et un domaine VSP4, et/ou le gène *FANCM* codant la protéine FANCM ayant au moins 30% d'identité de séquence, ou au moins 45% de similarité de séquence avec la protéine AtFANCM de séquence SEQ ID NO: 45, et contenant un domaine hélicase DEXDc et un domaine hélicase HELICc, ladite séquence d'ADN recombinant étant placée sous contrôle transcriptionnel d'un promoteur fonctionnel dans ladite plante ou une mutation dans le gène *TOP3A, FIDG* et/ou *FANCM,* ladite mutation induisant l'inhibition de la protéine TOP3A, FIDG et/ou FANCM dans ladite plante ; et/ou
- une séquence d'ADN recombinant codant pour une protéine TOP3A ayant au moins 50 % d'identité de séquence avec la protéine TOP3A de séquence SEQ ID NO: 2, comprenant un domaine TOPRIM et un domaine Topoisomérase IA non-altérés et au moins un domaine en doigt de zinc qui est inactivé, placée sous contrôle transcriptionnel d'un promoteur fonctionnel dans ladite plante.

L'invention englobe également des plantes génétiquement transformées par au moins une construction d'ADN de l'invention. De préférence, lesdites plantes sont des plantes transgéniques, dans laquelle la ou lesdites constructions sont contenues dans un transgène intégré dans le génome de la plante, de sorte qu'il est transmis aux générations successives de plantes. L'expression de la construction d'ADN de l'invention résulte en une régulation négative de l'expression du gène *RECQ4 et*/*ou TOP3A,* la régulation négative de l'expression du gène *TOP3A* étant combinée à l'expression d'une protéine TOP3A mutée en C-terminal, qui confère auxdites plantes transgéniques un taux de COs méiotiques supérieur à celui des plantes sauvages (ne contenant pas la construction d'ADN de l'invention) dont elles sont issues. Généralement ce taux de COs méiotiques est supérieur d'au moins 50%, de préférence au moins 2 fois supérieur à celui des plantes sauvages dont elles sont issues.

De manière particulièrement avantageuse, le taux de COs méiotiques peut encore être augmenté en combinant dans une même plante, l'inhibition de la protéine RECQ4 et /ou TOP3A avec celle de la protéine FANCM et/ou FIDG. Dans ce cas, le taux de COs méiotiques est au moins 3 fois supérieur, de préférence au moins 5 fois supérieur à celui des plantes sauvages dont elles sont issues.

L'augmentation du taux de COs méiotiques par inhibition de la protéine FANCM est décrite dans la Demande PCT WO2013038376.

La protéine FANCM est définie comme une protéine ayant au moins 30% et par ordre de préférence croissante, au moins 35, 40, 45, 50, 55, 60, 65, 70, 75, 80, 85, 90, 95 ou 98% d'identité de séquence, ou au moins 45%, et par ordre de préférence croissante, au moins 50, 55, 60, 65, 70, 75, 80, 85, 90, 95 ou 98% de similarité de séquence avec la protéine AtFANCM d'*Arabidopsis thaliana* (GenBank : NP_001185141 ; UniProtKB : F4HYE4), et contenant un domaine hélicase DEXDc (cd00046) et un domaine hélicase HELICc (cd00079). De préférence, le domaine hélicase DEXDc a au moins 65%, et par ordre de préférence croissante, au moins 70,75, 80, 85, 90, 95 ou 98% d'identité de séquence, ou au moins 75%, et par ordre de préférence croissante, au moins 80, 85, 90, 95 ou 98% de similarité de séquence avec le domaine DEXDc (acides aminés 129-272) de la protéine AtFANCM, et le domaine hélicase HELICc a au moins 60%, et par ordre de préférence croissante, au moins 65, 70, 75, 80, 85, 90, 95 ou 98% d'identité de séquence, ou au moins 70%, et par ordre de préférence croissante, au moins 75, 80, 85, 90, 95 ou 98% de similarité de séquence avec le domaine HELICc (acides aminés 445-570) de la protéine AtFANCM. La séquence polypeptidique de la protéine AtFANCM est représentée dans la liste de séquences en annexe sous le numéro SEQ ID NO: 45.

Les valeurs d'identité et de similarité de séquence indiquées pour la protéine FANCM sont calculées en utilisant le programme BLASTP ou le programme Needle avec les paramètres par défaut. Les calculs de similarité sont effectués en utilisant la matrice BLOSUM62.

La séquence de la protéine FIDGETIN-LIKE 1 d'*Arabidopsis thaliana* est représentée dans la liste de séquences en annexe sous le numéro SEQ ID NO: 46. Une recherche dans les bases de données de séquences a permis d'identifier des orthologues d'*AtFIDG* chez un large panel d'eucaryotes, et il très probable que cette protéine est conservée dans l'ensemble des plantes terrestres. Des exemples non limitatifs d'orthologues d'*AtFIDG* sont cités dans le tableau, ci-après.

**Tableau III : Séquences d'orthologues d'AtFIDG**

| **Plante** | **Numéro d'accès** |
|---|---|
| *Brachypodium distachyon* | Genbank : XM_003576467 |
| *Carica papaya* | Phytozome : evm.model.supercontig_171.22 |
| | PLAZA : CP00171G00260 |
| *Fragaria vesca* | Phytozome : mrna25885.1 |
| | PLAZA : FV6G37220 |
| *Glycin max* | Phytozome : Glyma19g18350.2 et Glyma05g14440.2 |
| | PLAZA : GM19G18350 et GM05G14440 |
| *Oryza sativa* | GenBank : ABA97741 |
| *Populus tricocarpa* | GenBank : POPTR_0001s33870 |
| *Ricinus communis* | Genbank : XM_002509479 |
| *Solanum lycopersicum* | Genbank : XM_004233540.1 |
| *Sorghum bicolor* | Genbank : XM_002442067.1 |
| *Theobroma cacao* | Phytozome : Thecc1EG017182t1 |
| | PLAZA : TC04G003320 |
| *Vitis vinifera* | GenBank : CBI21358 |
| *Zea mays* | GenBank : DAA54951 |
| *Hordeum vulgare* | Genbank : BAK02801. |
| *Triticum urartu* | Genbank : EMS65393.1. |

La protéine FIDG a au moins 35%, et par ordre de préférence croissante, au moins 40, 45, 50, 55, 60, 65, 70, 75, 80, 85, 90, 95 ou 98% d'identité de séquence, ou au moins 50%, et par ordre de préférence croissante, au moins 50, 55, 60, 65, 70, 75, 80, 85, 90, 95 ou 98% de similarité de séquence avec la protéine AtFIDG de séquence SEQ ID NO: 46, et contenant un domaine AAA-ATPase (PF00004) et un domaine VSP4 (PF09336).

De préférence, ladite protéine FIDG a au moins 70%, et par ordre de préférence croissante, au moins 75, 80, 85, 90, 95 ou 98% d'identité de séquence, ou au moins 80%, et par ordre de préférence croissante, au moins 85, 90, 95 ou 98% de similarité de séquence avec l'un quelconque des orthologues d'*AtFIDG* dont la liste est indiquée dans le Tableau III, ci-dessus.

Selon un mode de mise en œuvre préféré de la présente invention, le domaine AAA-ATPase de ladite protéine FIDG a au moins 60%, et par ordre de préférence croissante, au moins 65, 70, 75, 80, 85, 90, 95 ou 98% d'identité de séquence, ou au moins 70%, et par ordre de préférence croissante, au moins 75, 80, 85, 90, 95 ou 98% de similarité de séquence avec le domaine AAA-ATPase de la protéine AtFIDG (acides aminés 431-561 de la SEQ ID NO: 46).

Selon un autre mode de mise en œuvre préféré de la présente invention, le domaine VSP4 de ladite protéine FIDG a au moins 60%, et par ordre de préférence croissante, au moins 65, 70, 75, 80, 85, 90, 95 ou 98% d'identité de séquence, ou au moins 70%, et par ordre de préférence croissante, au moins 75, 80, 85, 90, 95 ou 98% de similarité de séquence avec le domaine VSP4 de la protéine AtFIDG (acides aminés 623-672 de la SEQ ID NO: 46).

Avantageusement, ladite protéine FIDG contient en outre un domaine de liaison à RAD51, ayant au moins 50%, et par ordre de préférence croissante, au moins 55, 60, 65, 70, 75, 80, 85, 90, 95 ou 98% d'identité de séquence, ou au moins 70%, et par ordre de préférence croissante, au moins 75, 80, 85, 90, 95 ou 98% de similarité de séquence avec les acides aminés 268-346 de la SEQ ID NO: 46.

Les valeurs d'identité et de similarité de séquence indiquées pour la protéine FIDG sont calculées sur la totalité de la longueur des séquences comparées, en utilisant l'algorithme d'alignement global de Needleman-Wunsch (programme Needle EMBOSS avec les paramètres par défaut). Les calculs de similarité sont effectués en utilisant la matrice BLOSUM62.

La présente invention peut s'appliquer notamment dans le domaine de l'amélioration des plantes, afin d'accélérer l'obtention de nouvelles variétés. Elle permet aussi de faciliter la recombinaison entre espèces apparentées, et donc l'introgression de caractères d'intérêt. Elle permet également d'accélérer l'établissement de cartes génétiques et les clonages positionnels.

La présente invention s'applique à un large éventail de plantes d'intérêt agronomique monocotylédones ou dicotylédones. A titre d'exemples non-limitatifs, on peut citer le colza, le tournesol, la pomme de terre, le maïs, le blé, l'orge, le seigle, le sorgo, le riz, le soja, le haricot, la carotte, la tomate, la courgette, le poivron, l'aubergine, le navet, l'oignon, le pois, le concombre, le poireau, l'artichaut, la betterave, le choux, le chou-fleur, les salades, l'endive, le melon, la pastèque, le fraisier, le pommier, le poirier, le prunier, le peuplier, la vigne, le coton, la rose, la tulipe, etc.

La présente invention sera mieux comprise à l'aide du complément de description qui va suivre, qui se réfère à des exemples non-limitatifs illustrant les effets de mutations du gène *AtRECQ4* ou *AtTOP3A,* seules ou combinées à des mutations du gène *FANCM,* sur la recombinaison méiotique et le taux de Cos, avec références aux dessins annexés dans lesquels :
- **figure 1** **: Arbre phylogénétique des protéines RECQ4 de plantes à fleurs.** L'analyse a été réalisée sur la plate-forme Phylogeny.fr selon les étapes suivantes : les séquences ont été alignées avec T-Coffee (v6.85) en utilisant les méthodes suivantes d'alignement par paires : les 10 meilleurs alignements locaux (Lalign_pair), un alignement global exact (slow_pair). Après l'alignement, les positions avec des espaces ont été supprimées de l'alignement. L'arbre phylogénétique a été reconstruit en utilisant la méthode du maximum de vraisemblance, mise en œuvre dans le programme PhyML v3.0 aLRT. Les séquences des protéines ont été collectées dans la base de données PLAZA (http://bioinformatics.psb.ugent.be/plaza/) et celle du phytozome (http://www.phytozome. net.): *At* : *Arabidopsis thaliana. Al Arabidopsis lyrata. Bra : Brassica rapa. Esa: Thellungiella halophila. Cp: Carica papaya. Tcacao : Theobroma cacao. Graimndii: Gossypium raimondii. VV: Vitis vinifera. Ppersica Prunus persica. RC: Ricinus communis. ME : Manihot esculenta. Solyc: Solanum lycopersicum. Eucgr : Eucalypsus grandis. Phvul:Phaseolus vulgaris. Glyma: Glycine max. OS: Oryza sativa. BD: Brachypodium distachyon. Si: Setaria italica. Zmays: Zea mays. Sbicolor: Sorghum bicolor.*
- **figure 2****. Alignement des TOPOISOMERASES 3a d'eucaryotes à l'aide de T-Coffee.** Les domaines conservés sont indiqués par une ligne verticale ou horizontale. *At*: *arabidopsis thaliana. Sc. Saccharomyces cerevisiea. Os Oryza sativa. Hs Homo sapiens. Ds Drosophila melanogaster. Coenorabditis elegans. Sp Schizosaccharomyces pombe.*
   L'alignement a été réaliséà l'aide de T-Coffee (http://toolkit.tuebingen.mpg.de/t coffee) avec les paramètres par défaut.

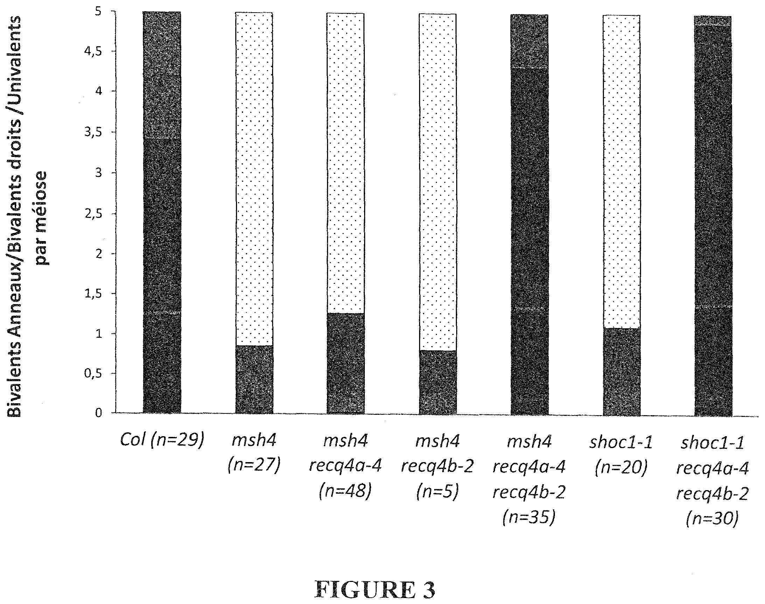

- **figure 3****. La double mutation de *RECQ4A* et *RECQ4B* restaure la** formation de bilents chez les mutants ***zmm.*** Nombre moyen de bivalents par méiocyte mâle. Bivalents en anneau (noir). Bivalents droits (gris). Paires d'univalents (points noir). Le nombre de cellules en métaphase I qui ont été analysées est indiqué entre parenthèses.

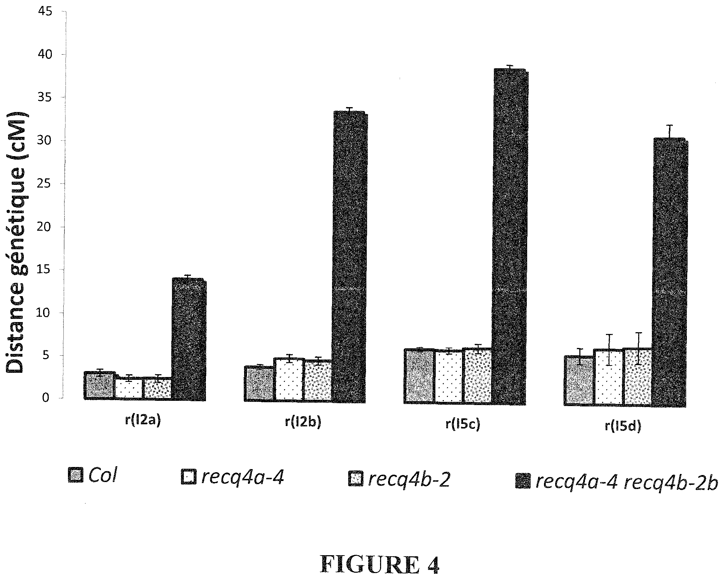

- **figure 4****. Les hélicases RECQ4A and RECQ4B limitent de façon redondante la formation de CO pendant la méiose.** Les distances génétiques dans 4 intervalles, mesurées à l'aide de lignées portant des marqueurs fluorescents, ont été calculées avec l'équation de Perkins et sont exprimées en centiMorgans. I2a et I2b sont des intervalles adjacents sur le chromosome 2, et I5c et I5d sur le chromosome 5.

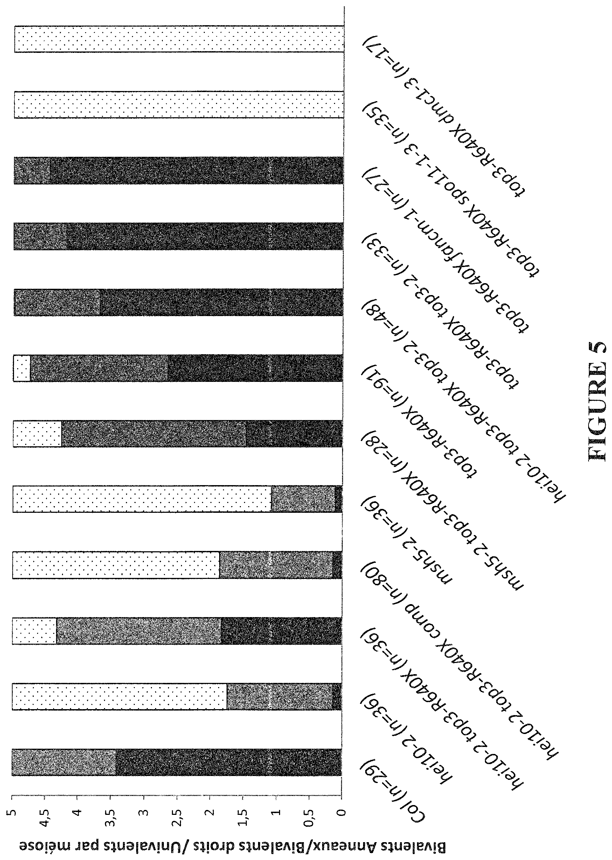

- **figure 5****. La mutation *top3a-R640X* restaure la formation** de **bivalents chez les mutants *zmm.*** Nombre moyen de bivalents par méiocyte mâle. Bivalents en anneau (noir). Bivalents droits (gris). Paires d'univalents (points noirs). Le nombre de cellules en métaphase I qui ont été analysées est indiqué entre parenthèses.

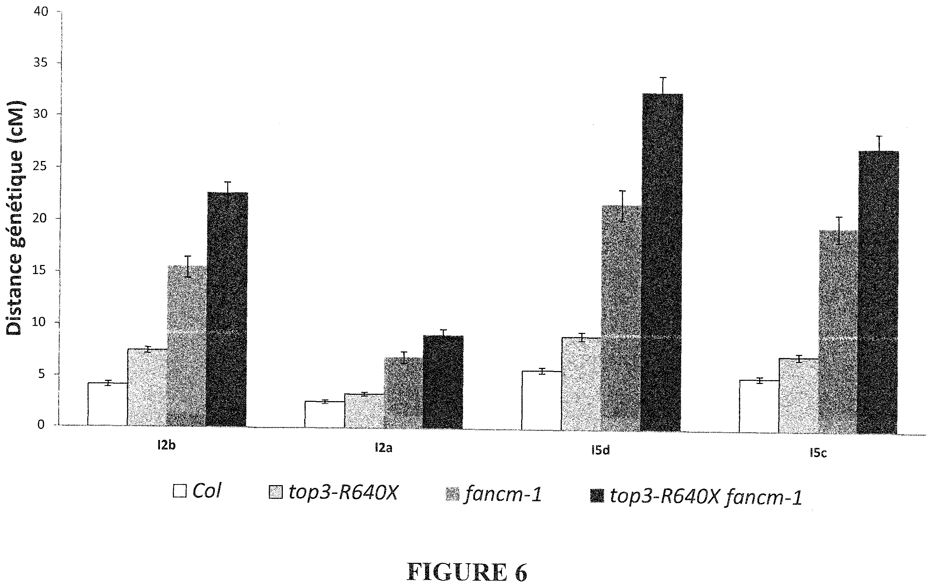

- **figure 6****. TOP3A et FANCM limitent de façon indépendante la formation de CO pendant la méiose.** Les distances génétiques dans 4 intervalles, mesurées à l'aide de lignées portant des marqueurs fluorescents, ont été calculées avec l'équation de Perkins et sont exprimées en centiMorgans. I2a et I2b sont des intervalles adjacents sur le chromosome 2, et I5c et I5d sur le chromosome 5.

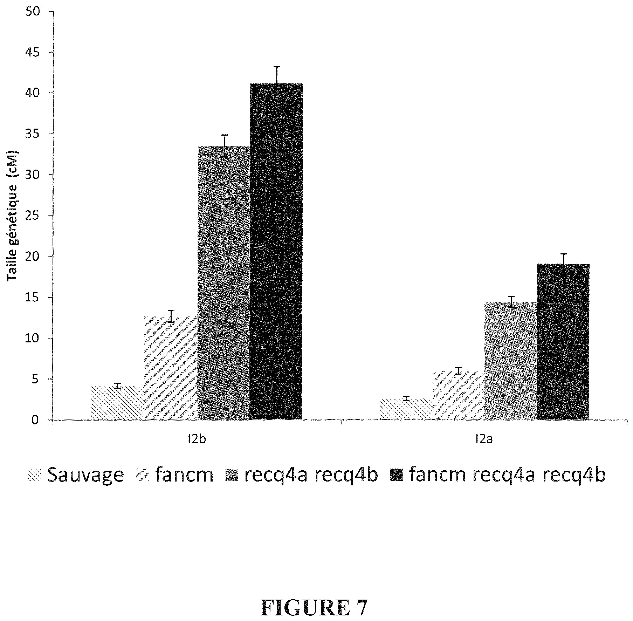

- **figure 7****. RECQ4 et FANCM limitent de façon indépendante la formation de CO pendant la méiose.** Les distances génétiques dans 2 intervalles, mesurées à l'aide de lignées portant des marqueurs fluorescents, ont été calculées avec l'équation de Perkins et sont exprimées en centiMorgans. I2a et I2b sont des intervalles adjacents sur le chromosome 2.

### EXEMPLE 1 : OBTENTION DE MUTANTS DU GENE RECQ4 SUPPRESSEURS DE MUTATIONS DE GENES ZMM

Des graines du mutant *msh4* d'*Arabidopsis thaliana* dans le fond génétique Landsberg eracta (cshl_GT14269 ; Drouaud et al., PLoS Genet., 2013, 9, e1003922; Higgins et al., Genes Dev., 2004, 18, 2557-2570) ont été mutées par EMS (éthylméthane sulfonate). Les plantes issues des graines mutées (population M1, hétérozygotes pour les mutations induites par l'EMS, et homozygotes pour la mutation du gène *ZMM*) ont un phénotype identique, résultant de l'inactivation du gène *ZMM,* qui se traduit par une diminution forte de la fréquence de COs, qui mène à une diminution forte du nombre de bivalents, et une baisse très importante de la fertilité (plantes « semi stériles »), résultant en la formation de siliques courtes qui se différencient facilement de celles des plantes sauvages. Les plantes M1 ont été auto-pollinisées, pour produire une population de descendants (population M2 ; environ 1000 familles) contenant potentiellement des plantes homozygotes pour les mutations induites par l'EMS. Des plantes de la population M2 ayant des siliques plus longues que celles des plantes *zmm* homozygotes de la génération M1 ont été sélectionnées et génotypées afin de vérifier leur statut homozygote pour la mutation *msh4.* Ce sont des suppresseurs.

Deux lignées de mutants suppresseurs de la mutation du gène *AtMSH4,* dénommées *msh4(s)84 et msh4(s)101,* font l'objet de cette étude. Le séquençage complet du génome de ces mutants a montré que les mutations correspondantes étaient localisées dans le gène *At1g10930* codant pour l'hélicase RECQ4A. Le mutant *msh4(s)84* comprend le changement C>T en position TAIR 10 : chrl :3652474 qui introduit un codon stop au niveau du codon W387. Le mutant *msh4(s)101* comprend le changement C>T en position TAIR 10 : chr1 :3650343 qui introduit la substitution G762D. Ces deux suppresseurs sont alléliques, démontrant que les mutations dans *RECQ4A* sont les mutations causales de la restauration de fertilité.

La comparaison de la séquence de l'écotype Landsberg (Gan et al., Nature, 2011, 477, 7365, 419-23) avec celle du génome de l'écotype Columbia a montré que leurs loci *RECQ4B* différaient par une série de polymorphismes de nucléotide unique et d'insertion/délétion, dont deux qui introduisent un codon stop prématuré (Q430>STOP) et un décalage de phase de lecture (Aa 501) chez Lansdberg. Ceci suggère fortement que le gène *RECQ4B* gène n'est pas fonctionnel chez Lansdberg.

Au vu de ces résultats, il a été émis l'hypothèse que la mutation *recq4a* chez Columbia n'était pas capable de compenser les effets de celle de *msh4* à cause de la redondance avec RECQ4B pendant la méiose. Ceci expliquerait la raison pour laquelle la mutation de *RECQ4A* seul, serait capable de compenser les effets de celle de *msh4* chez Landsberg mais pas Columbia.

Par conséquent, les mutations *msh4, recq4a,* et *recq4b* ont été combinées dans le fond génétique Columbia. Le nombre de bivalents en métaphase I du triple mutant *msh4 recq4a-4 recq4b-2* a été comparé avec celui de plantes sauvages (Col), de plantes *zmm* (*shoc1*/*Atzip2* ou *msh4*) et de doubles mutants *msh4 recq4a-4* et *msh4 recq4b-2.*

Les résultats sont illustrés par la Figure 3. Alors que les plantes sauvages montrent systématiquement 5 bivalents par méiose, les mutants *Atmsh4* montrent une forte diminution du nombre de bivalents, et donc l'apparition d'univalents. Chez les plantes sauvages, on observe une ségrégation normale des chromosomes, conduisant à la formation de gamètes équilibrés. En revanche, dans les mutants *zmm,* on observe une mauvaise ségrégation des chromosomes qui résulte d'une diminution du nombre de bivalents, induite par la mutation *zmm,* et qui conduit à des gamètes déséquilibrés, non-viables.

De plus, comme il a été montré précédemment (Higgins et al., The Plant Journal, 2011, 65, 492-502), les doubles mutants *msh4 recq4a-4* et *msh4 recq4b-2* sont quasi-stériles comme le mutant *msh4* et ont un nombre de bivalents en métaphase I, semblable à celui de *msh4* (Figure 3).

Au contraire, le triple mutant *msh4 recq4a-4 recq4b-2* est fertile et son nombre de bivalents est rétabli au niveau de celui du sauvage (Figure 3). De façon similaire, la double mutation *recq4a recq4b* est également capable de restaurer la fertilité et la formation de bivalents chez un autre mutant *zmm, shoc1*/*Atzip2* (Figure 3).

Ces résultats montrent que chez Columbia, *RECQ4A* et *RECQ4B* ont des fonctions redondantes qui empêchent la formation de COs méiotiques chez les mutants ***zmm.***

### EXEMPLE 2 : INFLUENCE DE L'INACTIVATION DES GENES RECQ4 SUR LA FREQUENCE DE RECOMBINAISON MEIOTIQUE

Les effets des mutations *recq4* sur la recombinaison méiotique ont ensuite été mesurés chez une plante sauvage (Columbia), *i.e,* en présence de *ZMMs* fonctionnels. La fréquence de recombinaison méiotique a été mesurée par l'analyse de tétrades à l'aide de lignées marquées avec un marqueur fluorescent, comme décrit par BERCHOWITZ & COPENHAVER, Nat. Protoc., 2008, 3, 41-50). La distance génétique a été mesurée sur 4 intervalles génétiques différents, 2 intervalles adjacents sur le chromosome 2 (I2a et I2b) et 2 intervalles adjacents sur le chromosome 5 (intervalles I5c et I5d). Les résultats sont illustrés par la Figure 4.

Chez les simples mutants *recq4a* et *recq4b,* les distances génétiques n'étaient pas significativement différentes du sauvage pour tous les intervalles testés (p>5%). Au contraire, les distances génétiques étaient considérablement augmentées chez le double mutant *recq4a recq4b* (p<10⁻⁹). Cette augmentation était d'un facteur moyen de 6,2 avec un écart de 4,6 à 8,6.

Ces résultats montrent que RECQ4A et RECQ4B jouent un rôle majeur redondant dans la limitation de la formation de COs pendant la méiose et constituent les facteurs génétiques anti-COs méiotiques les plus puissants identifiés à ce jour. De plus, ceci démontre que le fait d'avoir plus de 6 fois plus de COs que le sauvage n'a pas d'effets délétères sur l'intégrité des chromosomes, la ségrégation équilibrée des chromosomes, l'achèvement de la méiose et la fertilité.

### EXEMPLE 3 : OBTENTION DE MUTANTS DU GENE TOP3A SUPPRESSEURS DE MUTATIONS DE GENES ZMM

Un criblage similaire à celui réalisé pour *msh4* (exemple 1) a été effectué pour rechercher des suppresseurs du mutant *zmm hei10* (Chelysheva et al., PLoS Genet., 2012,8, e1002799). Parmi 1000 lignées mutagénisées issues du mutant *hei10-2,* un suppresseur, *hei10(s)61,* présentant une fertilité et un nombre de bivalents supérieur au mutant *hei10,* a été isolé (Figure 5). Le clonage de la mutation à partir de la carte génétique a défini un intervalle de 2,4 Mb sur le chromosome 5, entre les positions Tair10_ chr5:23.819.915 et Tair10_chr5:26.497.664. Le séquençage complet du génome de *hei10(s)61* a identifié une mutation non-sens à l'intérieur de cet intervalle, dans le gène *At5g63920* qui code pour la TOPOISOMERASE 3 alpha (TOP3A).

Ce gène semble être un bon candidat étant donné que TOP3A et Sgs1 appartiennent au complexe RTR, un complexe conservé qui est essentiel pour le maintien de l'intégrité du génome (Mankouri et Hickson, Trends Biochem Sci., 2007, 32, 538-46).

Afin de tester si cette mutation était la cause du phénotype, *hei10(s)61* a été transformé avec un clone génomique de 10kb contenant *TOP3A.* Tous les transformants présentaient le phénotype *d'hei10* (24 lignées indépendantes), étaient stériles et avaient 1,8 bivalents ± 0,75 en métaphase I (moyenne de 3 lignées indépendantes; Figure 5). Cela démontre que la mutation dans *TOP3A* était en fait la mutation causale qui a restauré la fertilité et la formation de bivalents chez le suppresseur *hei10(s)61.* Ceci montre qu'AtTOP3A empêche la formation de CO additionnels dans le contexte *hei10.* La mutation *hei10(s)61* change Arg640 en un codon stop (dénommée ci-après *top3a-R640X*)*.* Par conséquent, *top3a-R640X* code pour une protéine qui a des domaines TOPRIM et Topoisomerase intactes mais qui est tronquée de ces derniers 286 acides aminés qui contiennent les deux domaines en doigt de Zinc, prédits (Figure 2).

L'augmentation du nombre de bivalents et la restauration de fertilité observés chez le mutant *hei10 top3a-R640X* ont aussi été observés chez un double mutant *msh5 top3a-R640X (4,2* ± 0,9 bivalents par méiose ; Figure 5), montrant que la suppression observée n'était pas spécifique du fond génétique *hei10.*

Les plantes *hei10-2 top3a-2* et *hei10-2 top3a-R640X* diffèrent à la fois par leur phénotype somatique et méiotique. Comme un simple mutant *top3a-2, hei10-2 top3a-2* montre une croissance chétive et une stérilité complète, contrastant avec la croissance normale et la fertilité de *hei10-2 top3a-R640X.* En méiose, le double mutant *hei10 top3a-2* était indistinguable du simple mutant *top3a-2,* avec des structures aberrantes en métaphase I et une fragmentation massive en anaphase I. Chez *hei10-2 top3a-R640X,* les bivalents observés (4,2 par cellule, comparé à 1,7 chez *hei10-2,* figure 6), ségrégent en anaphase I sans fragmentation des chromosomes. Le phénotype *top3a-2* a montré que TOP3A est essentielle pour empêcher une catastrophe méiotique, alors que *top3a-R640X* semble être une séparation de fonction conduisant à la restauration de COs dans le fond génétique *hei10,* mais gardant son activité efficace de réparation. Cela suggère que TOP3A a une fonction double pendant la méiose, étant essentielle, à la fois pour résoudre les intermédiaires de recombinaison méiotique et aussi pour limiter la formation de COs.

Dans les plantes *hei10-2 top3a-R640X*/*top3a-2,* aucune fragmentation n'a été observée, montrant qu'une copie de *TOP3AR640X* est suffisante pour réparer les intermédiaires de recombinaison. Le nombre de bivalents dans ce fond génétique est même plus élevé que dans le double mutant *hei10 top3a-R640X* (4,9±0,1 vs 4,2±1,1 bivalents ±, T-test, figure 5), suggérant que la protéine TOP3A-R640X pourrait conserver de l'activité anti-CO.

Ensuite, l'effet de la mutation *top3a-R640X* chez des plantes possédant un *HEI10* fonctionnel, a été analysé. En accord avec les résultats obtenus dans le contexte *hei10,* les plantes *top3a-R640X* ne montrent aucune anomalie somatique et sont fertiles [graines par fruit: type sauvage=61 ±4 (n=40), *top3a-R640X =* 61±3 (n = 40)]. La méiose du mutant *top3a-R640X* est presque indistinguable de celle du sauvage avec aucune observation de fragmentation.

Toutefois, une fréquence faible d'univalents a été observée dans le simple mutant *top3a-R640X* (0,3 par cellule, Figure 5), alors que des univalents ne sont pas observés chez le sauvage. Cela suggère que le CO obligatoire est légèrement affecté dans le mutant *top3a-R640X.* Les plantes *top3a-R640X*/ *top3a-2* ne présentent aucune anomalie de développement, sont fertiles et aucun univalent n'a été observé (Figure 5).

### EXEMPLE 4 : INFLUENCE DE LA MUTATION DU GENE TOP3A SUR LA FREQUENCE DE RECOMBINAISON MEIOTIQUE

La fréquence de recombinaison méiotique du mutant *top3a-R640X a* ensuite été analysée par l'analyse de tétrades à l'aide de lignées marquées avec un marqueur fluorescent, dans 4 intervalles génétiques différents, comme décrit à l'exemple 2 (Figure 6). Dans tous les intervalles testés, les distances génétiques étaient augmentées, par comparaison avec le sauvage (P <10⁻⁵). Cette augmentation était en moyenne d'un facteur 1,5, avec un écart de 1,3 à 1,8. Cela montre que TOP3A a une activité anti-CO non seulement dans un contexte *hei10,* mais aussi dans des plantes sauvages pour *ZMMs.*

### EXEMPLE 5 : INFLUENCE DE L'INACTIVATION COMBINEE DES GENES TOP3A ET FANCM SUR LA FREQUENCE DE RECOMBINAISON MEIOTIQUE

L'hélicase FANCM a été le premier gène anti-CO méiotique décrit chez *Arabidopsis* ((Demande PCT WO 2013/038376 ; Crismani et al.,Science, 336, 6088, 1588-90). Pour tester si TOP3A et FANCM agissent dans la même voie, la fréquence de recombinaison a été mesurée chez le double mutant *top3a-R640Xfancm-1* (Figure 6). Dans les quatre intervalles testés, il a été observé que les distances génétiques étaient significativement plus grandes chez le double mutant *top3a-R640X fancm-1* que chez les deux simples mutants (p<10⁻⁵). L'augmentation de CO dans le double mutant, par comparaison au sauvage, était en moyenne d'un facteur 4,9, avec un écart de 3,5 à 5,6. Les effets des deux mutations sur la formation de CO sont cumulatifs, démontrant que FANCM et TOP3A agissent sur deux voies parallèles pour limiter la formation de CO pendant la méiose. De façon intéressante, le double mutant *top3a-R640X fancm-1* a une croissance normale, une méiose de type sauvage et est complètement fertile [graines par fruit: type sauvage =61 ±4 (n=40), *top3a-R640X fancm-1* =65 ±2 (n = 40)]. Cela confirme que le fait d'avoir cinq fois plus de COs que le sauvage, n'a pas d'effets immédiats délétères sur l'intégrité des chromosomes, la ségrégation équilibrée des chromosomes, l'achèvement de la méiose et la fertilité.

### EXEMPLE 6 : INFLUENCE DE L'INACTIVATION COMBINEE DES GENES FANCM ET RECQ4 SUR LA FREQUENCE DE RECOMBINAISON MEIOTIQUE

L'hélicase FANCM a été le premier gène anti-CO méiotique décrit chez *Arabidopsis* ((Demande PCT WO 2013/038376 ; Crismani et al.,Science, 336, 6088, 1588-90). Pour tester si RECQ4 et FANCM agissent dans la même voie, la fréquence de recombinaison a été mesurée chez le triple mutant *recq4a recq4b fancm-1* (Figure 7). Dans les deux intervalles testés, il a été observé que les distances génétiques étaient significativement plus grandes chez le triple mutant *recq4a recq4b fancm-1* que chez le double-mutant *recq4a recq4b* (p< 0.01) et le simple mutant *fancm-1* (p<10⁻⁶). L'augmentation de CO dans le triple mutant, par comparaison au sauvage, était en moyenne d'un facteur 9. Les effets des mutations des gènes *recq4* et *fancm* sur la formation de CO sont cumulatifs, démontrant que FANCM et RECQ4 agissent de façon parallèle pour limiter la formation de CO pendant la méiose. De façon intéressante, le triple mutant *recq4a recq4b fancm-1* a une croissance normale, une méiose de type sauvage (n=450) et est complètement fertile [graines par fruit: type sauvage =53,7 ±4,8 (n=40), *recq4a recq4b fancm-1*=49,2 ±5,3 (n = 40)]. Cela confirme que le fait d'avoir neuf fois plus de COs que le sauvage, n'a pas d'effets immédiats délétères sur l'intégrité des chromosomes, la ségrégation équilibrée des chromosomes, l'achèvement de la méiose et la fertilité.

## Revendications

1. Procédé pour augmenter la fréquence des crossovers méiotiques chez une plante, **caractérisé en ce qu'**il comprend l'inhibition dans ladite plante de l'expression ou de la fonction, d'au moins une protéine du complexe RTR dénommée RECQ4,
par mutagénèse du gène codant ladite protéine ou de son promoteur, par extinction du gène codant ladite protéine ou par un inhibiteur se liant spécifiquement à un domaine fonctionnel de ladite protéine,
ladite protéine RECQ4 ayant au moins 40 % d'identité de séquence avec la protéine RECQ4 de séquence SEQ ID NO: 1 et comprenant en outre une région ayant au moins 60% d'identité de séquence avec la région qui s'étend des positions 407 à 959 de la séquence SEQ ID NO : 1.

2. Procédé selon la revendication 1, **caractérisé en ce qu'**il comprend en outre l'inhibition dans ladite plante de l'expression ou de la fonction d'une protéine du complexe RTR dénommée TOP3A,
par mutagénèse du gène codant ladite protéine ou de son promoteur, par extinction du gène codant ladite protéine ou par un inhibiteur se liant spécifiquement à un domaine fonctionnel de ladite protéine,
ladite protéine TOP3A ayant au moins 50 % d'identité de séquence avec la protéine TOP3A de séquence SEQ ID NO: 2.

3. Procédé selon la revendication 1 ou la revendication 2, **caractérisé en ce qu'**il comprend en outre l'inhibition dans ladite plante de l'expression ou de la fonction , d'au moins une protéine choisie parmi :
- une protéine ci-après dénommée FIDG, ladite protéine ayant au moins 45% d'identité de séquence, ou au moins 60% de similarité de séquence avec la protéine AtFIDG de séquence SEQ ID NO: 46 et contenant un domaine AAA-ATPase et un domaine VSP4, et
- une protéine ci-après dénommée FANCM, ladite protéine ayant au moins 30% d'identité de séquence, ou au moins 45% de similarité de séquence avec la protéine AtFANCM de séquence SEQ ID NO: 45, et contenant un domaine hélicase DEXDc et un domaine hélicase HELICc,
par mutagénèse du gène codant ladite protéine ou de son promoteur, par extinction du gène codant ladite protéine ou par un inhibiteur se liant spécifiquement à un domaine fonctionnel de ladite protéine.

4. Procédé selon une quelconque des revendications 1 à 3, **caractérisé en ce que** l'inhibition de la protéine RECQ4 et/ou TOP3A et le cas échéant de la protéine FIDG et/ou FANCM, est obtenue par extinction du gène codant pour ladite protéine en exprimant dans ladite plante un ARN interférent ciblant ledit gène.

5. Cassette d'expression, **caractérisée en ce qu'**elle comprend une séquence d'ADN recombinant dont le transcrit est un ARN interférent ciblant le gène *RECQ4,* placée sous contrôle transcriptionnel d'un promoteur fonctionnel dans une cellule végétale, ledit gène *RECQ4* codant une protéine RECQ4 ayant au moins 40 % d'identité de séquence avec la protéine RECQ4 de séquence SEQ ID NO: 1 et comprenant en outre une région ayant au moins 60% d'identité de séquence avec la région qui s'étend des positions 407 à 959 de la séquence SEQ ID NO : 1.

6. Cassette d'expression selon la revendication 5, **caractérisée en ce qu'**elle comprend en outre une séquence d'ADN recombinant codant pour une protéine TOP3A comprenant un domaine TOPRIM et un domaine Topoisomérase IA non-altérés et au moins un domaine en doigt de zinc qui est inactivé, placée sous contrôle transcriptionnel d'un promoteur fonctionnel dans une cellule végétale, ladite protéine TOP3A ayant au moins 50 % d'identité de séquence avec la protéine TOP3A de séquence SEQ ID NO: 2.

7. Cassette d'expression selon la revendication 5 ou 6, **caractérisée en ce qu'**elle comprend en outre une séquence d'ADN recombinant dont le transcrit est un ARN interférent ciblant le gène *FANCM* ou *FIDG,* placée sous contrôle transcriptionnel d'un promoteur fonctionnel dans une cellule végétale, ledit gène *FANCM* codant une protéine FANCM ayant au moins 30% d'identité de séquence, ou au moins 45% de similarité de séquence avec la protéine AtFANCM de séquence SEQ ID NO: 45, et contenant un domaine hélicase DEXDc et un domaine hélicase HELICc et ledit gène *FIDG* codant une protéine FIDG ayant au moins 45% d'identité de séquence, ou au moins 60% de similarité de séquence avec la protéine AtFIDG de séquence SEQ ID NO: 46 et contenant un domaine AAA-ATPase et un domaine VSP4.

8. Vecteur recombinant contenant une cassette d'expression selon une quelconque des revendications 5 à 7.

9. Cellule-hôte transformée par un vecteur recombinant selon la revendication 8.

10. Plante mutante **caractérisée en ce qu'**elle contient une mutation dans le gène *RECQ4* codant une protéine RECQ4 ayant au moins 40 % d'identité de séquence avec la protéine RECQ4 de séquence SEQ ID NO: 1 et comprenant en outre une région ayant au moins 60% d'identité de séquence avec la région qui s'étend des positions 407 à 959 de la séquence SEQ ID NO : 1, ladite mutation induisant l'inhibition de l'expression ou de la fonction de la protéine RECQ4 dans ladite plante et étant choisie parmi les mutations ponctuelles ou la délétion de tout ou partie de la séquence codante ou du promoteur dudit gène.

11. Plante mutante selon la revendication 10, **caractérisée en ce qu'**elle contient en outre :
- une mutation dans le gène *TOP3A* codant une protéine TOP3A ayant au moins 50 % d'identité de séquence avec la protéine TOP3A de séquence SEQ ID NO: 2, le gène *FIDG* codant une protéine FIDG ayant au moins 45% d'identité de séquence, ou au moins 60% de similarité de séquence avec la protéine AtFIDG de séquence SEQ ID NO: 46 et contenant un domaine AAA-ATPase et un domaine VSP4, et/ou le gène *FANCM* codant une protéine FANCM ayant au moins 30% d'identité de séquence, ou au moins 45% de similarité de séquence avec la protéine AtFANCM de séquence SEQ ID NO: 45, et contenant un domaine hélicase DEXDc et un domaine hélicase HELICc, ladite mutation induisant l'inhibition de l'expression ou de la fonction de la protéine TOP3A, FIDG et/ou FANCM dans ladite plante ou au moins une séquence d'ADN recombinant dont le transcrit est un ARN interférent ciblant le gène *TOP3A, FIDG* ou *FANCM,* placée sous contrôle transcriptionnel d'un promoteur fonctionnel dans ladite plante ; et/ou
- une séquence d'ADN recombinant codant pour une protéine TOP3A ayant au moins 50 % d'identité de séquence avec la protéine TOP3A de séquence SEQ ID NO: 2, comprenant un domaine TOPRIM et un domaine Topoisomérase IA non-altérés et au moins un domaine en doigt de zinc qui est inactivé, placée sous contrôle transcriptionnel d'un promoteur fonctionnel dans ladite plante.

12. Plante transgénique contenant un transgène comprenant une séquence d'ADN recombinant dont le transcrit est un ARN interférent ciblant le gène *RECQ4,* placée sous contrôle transcriptionnel d'un promoteur fonctionnel dans ladite plante, ledit gène *RECQ4* codant une protéine RECQ4 ayant au moins 40 % d'identité de séquence avec la protéine RECQ4 de séquence SEQ ID NO: 1 et comprenant en outre une région ayant au moins 60% d'identité de séquence avec la région qui s'étend des positions 407 à 959 de la séquence SEQ ID NO : 1.

13. Plante transgénique selon la revendication 12, **caractérisée en ce qu'**elle contient en outre :
- une séquence d'ADN recombinant dont le transcrit est un ARN interférent ciblant le gène *TOP3A* codant la protéine TOP3A ayant au moins 50 % d'identité de séquence avec la protéine TOP3A de séquence SEQ ID NO: 2, le gène *FIDG* codant une protéine FIDG ayant au moins 45% d'identité de séquence, ou au moins 60% de similarité de séquence avec la protéine AtFIDG de séquence SEQ ID NO: 46 et contenant un domaine AAA-ATPase et un domaine VSP4, et/ou le gène *FANCM* codant la protéine FANCM ayant au moins 30% d'identité de séquence, ou au moins 45% de similarité de séquence avec la protéine AtFANCM de séquence SEQ ID NO: 45, et contenant un domaine hélicase DEXDc et un domaine hélicase HELICc, ladite séquence d'ADN recombinant étant placée sous contrôle transcriptionnel d'un promoteur fonctionnel dans ladite plante ou une mutation dans le gène *TOP3A, FIDG* et/ou *FANCM,* ladite mutation induisant l'inhibition de la protéine TOP3A, FIDG et/ou FANCM dans ladite plante ; et/ou
- une séquence d'ADN recombinant codant pour une protéine TOP3A ayant au moins 50 % d'identité de séquence avec la protéine TOP3A de séquence SEQ ID NO: 2, comprenant un domaine TOPRIM et un domaine Topoisomérase IA non-altérés et au moins un domaine en doigt de zinc qui est inactivé, placée sous contrôle transcriptionnel d'un promoteur fonctionnel dans ladite plante.

## Patentansprüche

1. Verfahren zum Erhöhen der Frequenz des meiotischen Crossovers bei einer Pflanze, **dadurch gekennzeichnet, dass** es die Inhibierung der Expression oder der Funktion von mindestens einem Protein des RECQ4 genannten RTR-Komplexes in der Pflanze umfasst,
durch Mutagenese des Gens, das das Protein kodiert, oder seines Promotors, durch Extinktion des Gens, das das Protein kodiert, oder durch einen Inhibitor, der sich spezifisch mit einer funktionalen Domäne des Proteins verbindet,
wobei das Protein RECQ4 mindestens 40 % Sequenzidentität mit dem Protein RECQ4 der Sequenz SEQ ID NO: 1 aufweist und weiter eine Region umfasst, die mindestens 60 % Sequenzidentität mit der Region aufweist, die sich von den Positionen 407 bis 959 der Sequenz SEQ ID NO: 1 erstreckt.

2. Verfahren nach Anspruch 1, **dadurch gekennzeichnet, dass** es weiter die Inhibierung der Expression oder der Funktion eines Proteins des TOP3A genannten RTR-Komplexes in der Pflanze umfasst,
durch Mutagenese des Gens, das das Protein kodiert, oder seines Promotors, durch Extinktion des Gens, das das Protein kodiert, oder durch einen Inhibitor, der sich spezifisch mit einer funktionalen Domäne des Proteins verbindet,
wobei das Protein TOP3A mindestens 50 % Sequenzidentität mit dem Protein TOP3A der Sequenz SEQ ID NO: 2 aufweist.

3. Verfahren nach Anspruch 1 oder Anspruch 2, **dadurch gekennzeichnet, dass** es weiter die Inhibierung der Expression oder der Funktion mindestens eines Proteins in der Pflanze umfasst, ausgewählt aus:
- einem hiernach FIDG genannten Protein, wobei das Protein mindestens 45 % Sequenzidentität oder mindestens 60 % Sequenzähnlichkeit mit dem Protein AtFIDG der Sequenz SEQ ID NO: 46 aufweist und eine AAA-ATPase-Domäne und eine VSP4-Domäne enthält, und
- einem hiernach FANCM genannten Protein, wobei das Protein mindestens 30 % Sequenzidentität oder mindestens 45 % Sequenzähnlichkeit mit dem Protein AtFANCM der Sequenz SEQ ID NO: 45 aufweist und eine DEXDc-Helikase-Domäne und eine HELICc-Helikase-Domäne enthält,
durch Mutagenese des Gens, das das Protein kodiert, oder seines Promotors, durch Extinktion des Gens, das das Protein kodiert, oder durch einen Inhibitor, der sich spezifisch mit einer funktionalen Domäne des Proteins verbindet.

4. Verfahren nach einem der Ansprüche 1 bis 3, **dadurch gekennzeichnet, dass** die Inhibierung des Proteins RECQ4 und/oder TOP3A und soweit erforderlich des Proteins FIDG und/oder FANCM durch Extinktion des Gens erhalten wird, das das Protein kodiert, indem in der Pflanze eine interferierende RNA exprimiert wird, die auf das Gen abzielt.

5. Expressionskassette, **dadurch gekennzeichnet, dass** sie eine rekombinante DNA-Sequenz umfasst, von der das Transkript eine interferierende RNA ist, die auf das Gen *RECQ4* abzielt, das unter transkriptionaler Kontrolle eines funktionalen Promotors in einer pflanzlichen Zelle platziert ist, wobei das Gen RECQ4 ein Protein RECQ4 kodiert, das mindestens 40 % Sequenzidentität mit dem Protein RECQ4 der Sequenz SEQ ID NO: 1 aufweist und weiter eine Region umfasst, die mindestens 60 % Sequenzidentität mit der Region aufweist, die sich von Positionen 407 bis 959 der Sequenz SEQ ID NO: 1 erstreckt.

6. Expressionskassette nach Anspruch 5, **dadurch gekennzeichnet, dass** sie weiter eine rekombinante DNA-Sequenz umfasst, die für ein Protein TOP3A kodiert, das eine TOPRIM-Domäne und eine Topoisomerase-IA-Domäne, die nicht verändert sind, und mindestens eine Zinkfinger-Domäne umfasst, die inaktiviert ist, die unter transkriptionaler Kontrolle eines funktionalen Promotors in einer pflanzlichen Zelle platziert ist, wobei das Protein TOP3A mindestens 50 % Sequenzidentität mit dem Protein TOP3A der Sequenz SEQ NO ID: 2 aufweist.

7. Expressionskassette nach Anspruch 5 oder 6, **dadurch gekennzeichnet, dass** sie weiter eine rekombinante DNA-Sequenz, von der das Transkript eine interferierende RNA ist, die auf das Gen *FANCM* oder *FIDG* abzielt, das unter transkriptionaler Kontrolle eines funktionalen Promotors in einer pflanzlichen Zelle platziert ist, wobei das Gen *FANCM,* das ein Protein FANCM kodiert, das mindestens 30 % Sequenzidentität oder mindestens 45 % Sequenzähnlichkeit mit dem Protein AtFANCM der Sequenz SEQ ID NO: 45 aufweist und eine DEXDc-Helikase-Domäne und eine HELICc-Helikase-Domäne enthält, und das Gen *FIDG* umfasst, das ein Protein FIDG kodiert, das mindestens 45 % Sequenzidentität oder mindestens 60 % Sequenzähnlichkeit mit dem Protein AtFIDG der Sequenz SEQ ID NO: 46 aufweist und eine AAA-ATPase-Domäne und eine VSP4-Domäne enthält.

8. Rekombinanter Vektor, der eine Expressionskassette nach einem der Ansprüche 5 bis 7 enthält.

9. Wirtszelle, die durch einen rekombinanten Vektor nach Anspruch 8 transformiert ist.

10. Mutante Pflanze, **dadurch gekennzeichnet, dass** sie eine Mutation in dem Gen *RECQ4* enthält, das ein Protein RECQ4 kodiert, das mindestens 40 % Sequenzidentität mit dem Protein RECA4 der Sequenz SEQ ID NO: 1 aufweist und weiter eine Region umfasst, die mindestens 60 % Sequenzidentität mit der Region aufweist, die sich von Positionen 407 bis 959 der Sequenz SEQ ID NO: 1 erstreckt, wobei die Mutation die Inhibierung der Expression oder der Funktion des Proteins RECQ4 in der Pflanze induziert und ausgewählt ist aus Punktmutationen oder Deletion von allem oder eines Teils der kodierenden Sequenz oder des Promotors des Gens.

11. Mutante Pflanze nach Anspruch 10, **dadurch gekennzeichnet, dass** sie ferner Folgendes enthält:
- eine Mutation in dem Gen *TOP3A,* das ein Protein TOP3A kodiert, das mindestens 50 % Sequenzidentität mit dem Protein TOP3A der Sequenz SEQ ID NO: 2 aufweist, wobei das Gen FIDG ein Protein FIDG kodiert, das mindestens 45 % Sequenzidentität oder mindestens 60 % Sequenzähnlichkeit mit dem Protein AtFIDG von Sequenz SEQ ID NO: 46 aufweist und eine AAA-ATPase-Domäne und eine VSP4-Domäne enthält, und/oder das Gen *FANCM,* das ein Protein FANCM kodiert, das mindestens 30 % Sequenzidentität oder mindestens 45 Sequenzähnlichkeit mit dem Protein AtFANCM von Sequenz SEQ ID NO: 45 aufweist und eine DEXDc-Helikase-Domäne und eine HELICc-Helikase-Domäne, wobei die Mutation die Inhibierung der Expression oder der Funktion des Proteins TOP3A, FIDG und/oder FANCM in der pflanzlichen Zelle induziert, oder mindestens eine rekombinante DNA-Sequenz enthält, von der das Transkript eine interferierende RNA ist, die auf das Gen *TOP3A, FIDG* oder *FANCM* abzielt, die unter transkriptionaler Kontrolle eine funktionalen Promotors in der Pflanze platziert ist; und/oder
- eine rekombinante DNA-Sequenz, die für ein Protein TOP3A kodiert, das mindestens 50 % Sequenzidentität mit dem Protein TOP3A der Sequenz SEQ NO ID: 2 aufweist, umfassend eine TOPRIM-Domäne und eine Topoisomerase-IA-Domäne, die nicht verändert sind, und mindestens eine Zinkfinger-Domäne, die inaktiviert ist, die unter transkriptionaler Kontrolle eines funktionalen Promotors in der Pflanze platziert ist.

12. Transgene Pflanze, die ein Transgen enthält, das eine rekombinante DNA-Sequenz umfasst, von der das Transkript eine interferierende RNA ist, die auf das Gen *RECQ4* abzielt, das unter transkriptionaler Kontrolle eines funktionalen Promotors in der Pflanze platziert ist, wobei das Gen *RECQ4* ein Protein RECQ4 kodiert, das mindestens 40 % Sequenzidentität mit dem Protein RECQ4 der Sequenz SEQ ID NO: 1 aufweist und weiter eine Region umfasst, die mindestens 60 % Sequenzidentität mit der Region aufweist, die sich von Positionen 407 bis 959 der Sequenz SEQ ID NO: 1 erstreckt.

13. Transgene Pflanze nach Anspruch 12, **dadurch gekennzeichnet, dass** sie ferner Folgendes enthält:
- eine rekombinante DNA-Sequenz, von der das Transkript eine interferierende RNA ist, die auf das Gen *TOP3A* abzielt, das das Protein TOP3A kodiert, das mindestens 50 % Sequenzidentität mit dem Protein TOP3A der Sequenz SEQ ID NO: 2 aufweist, wobei das Gen *FIDG* ein Protein FIDG kodiert, das mindestens 45 % Sequenzidentität oder mindestens 60 % Sequenzähnlichkeit mit dem Protein AtFIDG der Sequenz SEQ ID NO: 46 aufweist und eine AAA-ATPase-Domäne und eine VSP4-Domäne enthält, und/oder das Gen *FANCM,* das das Protein FANCM kodiert, das mindestens 30 % Sequenzidentität oder mindestens 45 Sequenzähnlichkeit mit dem Protein AtFANCM der Sequenz SEQ ID NO: 45 aufweist und eine DEXDc-Helikase-Domäne und eine HELICc-Helikase-Domäne enthält, wobei die rekombinante DNA-Sequenz unter transkriptionaler Kontrolle eines funktionalen Promotors in der Pflanze platziert ist oder eine Mutation in dem Gen *TOP3A, FIDG* und/oder *FANCM,* wobei die Mutation die Inhibierung des Proteins TOP3A, FIDG und/oder FANCM in der Pflanze induziert; und/oder
- eine rekombinante DNA-Sequenz, die für ein Protein TOP3A kodiert, das mindestens 50 % Sequenzidentität mit dem Protein TOP3A der Sequenz SEQ NO ID: 2 aufweist, umfassend eine TOPRIM-Domäne und eine Topoisomerase-IA-Domäne, die nicht verändert sind, und mindestens eine Zinkfinger-Domäne, die inaktiviert ist, die unter transkriptionaler Kontrolle eines funktionalen Promotors in der Pflanze platziert ist.

## Claims

1. A method for increasing the frequency of meiotic crossovers in a plant, **characterized in that** it comprises inhibiting in said plant the expression or function of at least one protein of the RTR complex called RECQ4,
by mutagenesis of the gene encoding said protein or its promoter, by extinction of the gene encoding said protein, or by an inhibitor specifically binding to a functional domain of said protein,
said RECQ4 protein having at least 40% sequence identity with the RECQ4 protein of sequence SEQ ID NO: 1 and further comprising a region having at least 60% sequence identity with the region extending from positions 407 to 959 of the sequence SEQ ID NO: 1.

2. The method according to claim 1, **characterized in that** it further comprises inhibiting in said plant the expression or function of a protein of the RTR complex called TOP3A, by mutagenesis of the gene encoding said protein or its promoter, by extinction of the gene encoding said protein, or by an inhibitor specifically binding to a functional domain of said protein,
said TOP3A protein having at least 50% sequence identity with the TOP3A protein of sequence SEQ ID NO: 2.

3. The method according to claim 1 or claim 2, **characterized in that** it further comprises inhibiting in said plant the expression or function of at least one protein selected from:
- a protein hereinafter referred to as FIDG, said protein having at least 45% sequence identity, or at least 60% sequence similarity, with the AtFIDG protein of sequence SEQ ID NO: 46 and containing an AAA-ATPase domain and a VSP4 domain, and
- a protein hereinafter referred to as FANCM, said protein having at least 30% sequence identity, or at least 45% sequence similarity with the AtFANCM protein of sequence SEQ ID NO: 45, and containing a DEXDc helicase domain and a HELICc helicase domain,
by mutagenesis of the gene encoding said protein or its promoter, by extinction of the gene encoding said protein, or by an inhibitor specifically binding to a functional domain of said protein.

4. The method according to any one of claims 1 to 3, **characterized in that** the inhibition of the RECQ4 and/or TOP3A protein and, where applicable, the FIDG and/or FANCM protein, is obtained by silencing the gene encoding said protein by expressing in said plant an interfering RNA targeting said gene.

5. An expression cassette, **characterized in that** it comprises a recombinant DNA sequence whose transcript is an interfering RNA targeting the RECQ4 gene, placed under the transcriptional control of a functional promoter in a plant cell, said RECQ4 gene encoding a RECQ4 protein having at least 40% sequence identity with the RECQ4 protein of sequence SEQ ID NO: 1 and further comprising a region having at least 60% sequence identity with the region extending from positions 407 to 959 of the sequence SEQ ID NO: 1.

6. The expression cassette according to claim 5, **characterized in that** it further comprises a recombinant DNA sequence encoding a TOP3A protein comprising an unaltered TOPRIM domain and a Topoisomerase IA domain and at least one zinc finger domain that is inactivated, placed under the transcriptional control of a functional promoter in a plant cell, said TOP3A protein having at least 50% sequence identity with the TOP3A protein of sequence SEQ ID NO: 2.

7. The expression cassette according to claim 5 or 6, **characterized in that** it further comprises a recombinant DNA sequence whose transcript is an interfering RNA targeting the *FANCM* or *FIDG* gene, placed under the transcriptional control of a functional promoter in a plant cell, said *FANCM* gene encoding a FANCM protein having at least 30% sequence identity, or at least 45% sequence similarity, with the AtFANCM protein of sequence SEQ ID NO: 45, and containing a DEXDc helicase domain and a HELICc helicase domain, and said *FIDG* gene encoding a FIDG protein having at least 45% sequence identity, or at least 60% sequence similarity, with the AtFIDG protein of sequence SEQ ID NO: 46 and containing an AAA-ATPase domain and a VSP4 domain.

8. A recombinant vector containing an expression cassette according to any one of claims 5 to 7.

9. A host cell transformed with a recombinant vector according to claim 8.

10. A mutant plant **characterized in that** it contains a mutation in the RECQ4 gene encoding a RECQ4 protein having at least 40% sequence identity with the RECQ4 protein of sequence SEQ ID NO: 1 and further comprising a region having at least 60% sequence identity with the region extending from positions 407 to 959 of the sequence SEQ ID NO: 1, said mutation inducing the inhibition of the expression or function of the RECQ4 protein in said plant and being selected from the point mutations or deletion of all or part of the coding sequence or the promoter of said gene.

11. The mutant plant according to claim 10, **characterized in that** it further contains:
- a mutation in the *TOP3A* gene encoding a TOP3A protein having at least 50% sequence identity with the TOP3A protein of sequence SEQ ID NO: 2, the *FIDG* gene encoding an FIDG protein having at least 45% sequence identity, or at least 60% sequence similarity, with the AtFIDG protein of sequence SEQ ID NO: 46 and containing an AAA-ATPase domain and a VSP4 domain, and/or the *FANCM* gene encoding a FANCM protein having at least 30% sequence identity, or at least 45% sequence similarity, with the AtFANCM protein of sequence SEQ ID NO: 45, and containing a DEXDc helicase domain and a HELICc helicase domain, said mutation inducing the inhibition of the expression or function of the TOP3A, FIDG and/or FANCM protein in said plant, or at least one recombinant DNA sequence whose transcript is an interfering RNA targeting the *TOP3A, FIDG,* or *FANCM* gene*,* placed under the transcriptional control of a functional promoter in said plant; and/or
- a recombinant DNA sequence encoding a TOP3A protein having at least 50% sequence identity with the TOP3A protein of sequence SEQ ID NO: 2, comprising an unaltered TOPRIM domain and a non-altered Topoisomerase IA domain and at least one zinc finger domain that is inactivated, placed under the transcriptional control of a functional promoter in said plant.

12. A transgenic plant containing a transgene comprising a recombinant DNA sequence whose transcript is an interfering RNA targeting the *RECQ4* gene, placed under the transcriptional control of a functional promoter in said plant, said *RECQ4* gene encoding a RECQ4 protein having at least 40% sequence identity with the RECQ4 protein of sequence SEQ ID NO: 1 and further comprising a region having at least 60% sequence identity with the region extending from positions 407 to 959 of sequence SEQ ID NO: 1.

13. The transgenic plant according to claim 12, **characterized in that** it further contains:
- a recombinant DNA sequence whose transcript is an interfering RNA targeting the *TOP3A* gene encoding the TOP3A protein having at least 50% sequence identity with the TOP3A protein of sequence SEQ ID NO: 2, the *FIDG* gene encoding an FIDG protein having at least 45% sequence identity, or at least 60% sequence similarity, with the AtFIDG protein of sequence SEQ ID NO: 46 and containing an AAA-ATPase domain and a VSP4 domain, and/or the *FANCM* gene encoding the FANCM protein having at least 30% sequence identity, or at least 45% sequence similarity with the AtFANCM protein of sequence SEQ ID NO: 45, and containing a DEXDc helicase domain and a HELICc helicase domain, said recombinant DNA sequence being placed under the transcriptional control of a functional promoter in said plant, or a mutation in the *TOP3A, FIDG* and/or *FANCM* gene, said mutation inducing the inhibition of the TOP3A, FIDG and/or FANCM protein in said plant; and/or
- a recombinant DNA sequence encoding a TOP3A protein having at least 50% sequence identity to the TOP3A protein of sequence SEQ ID NO: 2, comprising an unaltered TOPRIM domain and a non-altered Topoisomerase IA domain and at least one zinc finger domain that is inactivated, placed under the transcriptional control of a functional promoter in said plant.
